(19) Europäisches Patentamt
European Patent Office
Office européen des brevets

(11) Veröffentlichungsnummer: **0 006 524**
**A1**

# (12) EUROPÄISCHE PATENTANMELDUNG

(21) Anmeldenummer: 79101887.2

(22) Anmeldetag: 11.06.79

(51) Int. Cl.[3]: C 07 D 405/04, A 61 K 31/445

(30) Priorität: 22.06.78 CH 6823/78

(43) Veröffentlichungstag der Anmeldung: 09.01.80 Patentblatt 80/1

(84) Benannte Vertragsstaaten: AT BE CH DE FR GB IT LU NL SE

(71) Anmelder: CIBA-GEIGY AG, Patentabteilung Postfach, CH-4002 Basel (CH)

(72) Erfinder: Paioni, Romeo, Dr., Thiersteinerstrasse 22, CH-4153 Reinach (CH)
Erfinder: Schilling, Walter, Dr., Im Muspenacker, CH-4249 Himmelried (CH)
Erfinder: Bernasconi, Raymond, Dr., Amselstrasse 17A, CH-4104 Oberwil (CH)

## (54) Neue Tetrahydropyridin- und Piperidinderivate und deren Säureadditionssalze, Verfahren zu deren Herstellung und solche enthaltende pharmazeutische Zusammensetzungen.

(57) Die vorliegende Erfindung betrifft neue Tetrahydropyridin- und Piperidinderivate der allgemeinen Formel

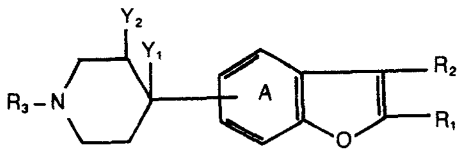

(I)

in welcher $R_1$ und $R_2$ unabhängig voneinander Wasserstoff oder Niederalkyl oder zusammen Niederalkylen, $R_3$ Wasserstoff, Niederalkyl, Niederalkenyl, Niederalkinyl, Cycloalkyl oder Cycloalkylniederalkyl und $Y_1$ und $Y_2$ je Wasserstoff oder zusammen eine zusätzliche Bindung bedeuten und der Ring A nicht weiter substituiert oder weiter substituiert ist, sowie deren Säureadditionssalze mit antidepressiver Wirksamkeit, Verfahren zur Herstellung dieser Verbindungen und ihrer Säureadditionssalze und solche enthaltende pharmazeutische Zusammensetzungen.

EP 0 006 524 A1

0006524

CIBA-GEIGY AG
Basel, Schweiz

Case 4-11773/+

Neue Tetrahydropyridin- und Piperidinderivate und deren Säureadditionssalze, Verfahren zu deren Herstellung und solche enthaltende pharmazeutische Zusammensetzungen

Die vorliegende Erfindung betrifft neue Tetrahydropyridin- und Piperidinderivate mit wertvollen pharmakologischen Eigenschaften, Verfahren zu deren Herstellung, diese Verbindungen enthaltende pharmazeutische Präparate und deren Anwendung.

Die erfindungsgemässen Tetrahydropyridin- und Piperidinderivate entsprechen der allgemeinen Formel I

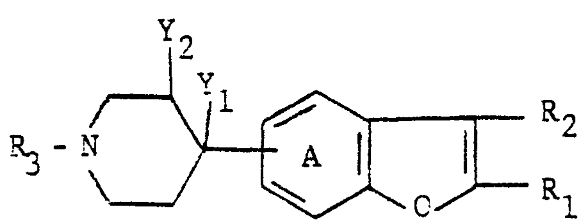

(I)

in welcher $R_1$ und $R_2$ unabhängig voneinander Wasserstoff oder Niederalkyl oder zusammen Niederalkylen, $R_3$ Wasserstoff, Niederalkyl, Niederalkenyl, Niederalkinyl, Cycloalkyl oder Cycloalkylniederalkyl und $Y_1$ und $Y_2$ je Wasserstoff oder zusammen eine zusätzliche Bindung bedeuten und der Ring A nicht weiter substituiert oder weiter substituiert ist.

Ebenfalls zum Gegenstand der Erfindung gehören die Säureadditionssalze, insbesondere die pharmazeutisch annehmbaren Säureadditionssalze der Verbindungen der allgemeinen Formel I und deren Herstellung.

Vor- und nachstehend werden unter "niederen" organischen Verbindungen und von diesen abgeleiteten Resten insbesondere solche Verbindungen und Reste verstanden, die bis zu 7, vor allem bis zu 4, Kohlenstoffatome aufweisen.

Der Ring A kann als weitere Substituenten beispielsweise Niederalkyl, Niederalkoxy, Halogen bis Atomnummer 35, Cyano, Hydroxy oder Trifluormethyl enthalten, wobei die drei letztgenannten Reste vorzugsweise höchstens einmal und die übrigen höchstens dreimal, vorzugsweise höchstens zweimal und insbesondere höchstens einmal vorkommen. Die Gesamtzahl der weiteren Substituenten des Ringes A kann drei betragen, vorzugsweise liegen jedoch nicht mehr als zwei weitere Substituenten vor. Insbesondere betrifft die Erfindung Verbindungen der allgemeinen Formel I, in denen der Ring A einen oder keinen weiteren Substituenten enthält.

Niederalkyl als $R_1$, $R_2$ und Substituent des Ringes A enthält beispielsweise bis zu 7, vor allem bis zu 4, Kohlenstoffatome und kann verzweigt sowie in beliebiger Stellung gebunden sein, ist aber vorzugsweise geradkettig. Als Beispiele seien vor allem Butyl, Isobutyl, Tert.butyl, Propyl, Isopropyl und speziell Aethyl und Methyl genannt.

Durch $R_1$ und $R_2$ zusammen gebildetes Niederalkylen kann 3 bis 7, vorzugsweise 3 bis 5 Kohlenstoffatome enthalten und ist z.B. Trimethylen, Pentamethylen und vor allem Tetramethylen.

Niederalkoxy als Substituent des Ringes A enthält beispielsweise bis zu 7, vor allem bis zu 4 Kohlenstoffatome und kann verzweigt sein, wobei der Sauerstoff in beliebiger Stellung gebunden sein kann, ist aber vorzugsweise geradkettig. Als Beispiele seien Butoxy, Propoxy, Isopropoxy, Aethoxy und insbesondere Methoxy genannt.

Halogen als Substituent des Ringes A

ist beispielsweise Halogen bis und mit Atomnummer 35, d.h. Fluor, Chlor oder Brom.

Niederalkenyl $R_3$ enthält höchstens 7 und insbesondere 3 bis 5 Kohlenstoffatome, wobei sich ebenso wie im Niederalkinyl $R_3$ die Mehrfachbindung vorzugsweise nicht an dem mit dem Stickstoffatom verbundenen Kohlenstoffatom befindet. Beispielsweise ist Niederalkenyl $R_3$ 1-Methylallyl, 3-Methyl-2-butenyl, insbesondere 2-Butenyl oder 2-Methylallyl und in erster Linie Allyl. Niederalkinyl $R_3$ enthält höchstens 7 und vorzugsweise 3 bis 4 Kohlenstoffatome und ist z.B. 2-Butinyl und insbesondere 2-Propinyl.

Cycloalkyl $R_3$ enthält vorzugsweise 3 bis 7 Kohlenstoffatome und ist beispielsweise Cyclopentyl, Cyclohexyl, Cycloheptyl und vor allem Cyclopropyl.

Cycloalkylniederalkyl $R_3$ enthält vorzugsweise 4 bis 9 Kohlenstoffatome und ist beispielsweise Cyclopropyläthyl, Cyclobutylmethyl, Cyclopentylmethyl, Cyclopentyläthyl, Cyclohexylmethyl, Cyclohexyläthyl, Cycloheptylmethyl oder Cycloheptyläthyl und insbesondere Cyclopropylmethyl.

Die Verbindungen der allgemeinen Formel I und ihre pharmazeutisch annehmbaren Säureadditionssalze besitzen wertvolle pharmakologische Eigenschaften. So zeigen sie eine ausgeprägte Hemmwirkung auf die Aufnahme von Serotonin durch Mittelhirnsynaptosomen, die beispielsweise an der Ratte bei oraler Verabreichung von Dosen von etwa 3 bis etwa 100 mg/kg nachgewiesen werden kann. Sie hemmen auch in gleicher Dosierung die durch H 75/12 induzierte Serotonin-Depletion im Rattenhirn. Weiter hemmen sie die Aufnahme von Noradrenalin durch Mittelhirnsynaptosomen von Ratten und die durch H 77/77' induzierte Noradrenalin-Depletion im Rattenhirn bei oraler Verabreichung in Dosen von etwa 3 bis etwa 100 mg/kg. In der gleichen Dosierung bewirken sie auch eine Mono-

aminoxidase-Hemmung im Rattenhirn und in der Rattenleber, die unter Verwendung von $^{14}C$-Serotonin und $^{14}C$-Phenäthylamin als Substrate nachgewiesen werden kann. Bei intraperitonealer Applikation an der Maus in Dosen von etwa 3 bis etwa 100 mg/kg potenzieren sie ferner den durch 5-Hydroxytryptophan induzierten "head-twitch". Ueberdies zeichnen sie sich durch gute Verträglichkeit aus. Die neuen Verbindungen der allgemeinen Formel I und ihre pharmazeutisch annehmbaren Säureadditionssalze können daher als Psychopharmaka, insbesondere als Antidepressiva, beispielsweise zur Behandlung von Gemütsdepressionen, Anwendung finden.

Die Erfindung betrifft insbesondere Verbindungen der allgemeinen Formel I, in denen $R_1$, $R_2$, $R_3$, $Y_1$ und $Y_2$ die unter der Formel I angegebene Bedeutung haben, und Ring A nicht weiter substituiert oder durch Niederalkyl, Niederalkoxy, Halogen bis Atomnummer 35, Cyano oder Hydroxy einfach weiter substituiert ist, und deren Säureadditionssalze. Vor allem betrifft die Erfindung Verbindungen der allgemeinen Formel I, in denen $R_1$ Methyl, $R_2$ Wasserstoff oder Methyl, oder $R_1$ und $R_2$ zusammen Tetramethylen bedeuten, $R_3$, $Y_1$ und $Y_2$ die unter der Formel I angegebene Bedeutung haben, und Ring A nicht weiter substituiert oder durch Niederalkyl, Niederalkoxy, Halogen bis Atomnummer 35, Cyano oder Hydroxy einfach weitersubstituiert ist, und deren Säureadditionssalze. Ganz besonders betrifft die Erfindung Verbindungen der allgemeinen Formel I, in denen $R_1$ Methyl, $R_2$ Methyl oder Wasserstoff, oder $R_1$ und $R_2$ zusammen Tetramethylen bedeuten, $R_3$ die unter der Formel I angegebene Bedeutung hat, indessen vorzugsweise Wasserstoff oder definitionsgemäss Reste mit höchstens 4 Kohlenstoffatomen, und zwar als Niederalkenyl insbesondere Allyl, als Niederalkinyl insbesondere 2-Propinyl, als Cycloalkyl insbesondere Cyclopropyl und als Cycloalkylniederalkyl insbesondere Cyclopropylmethyl bedeutet, $Y_1$ und $Y_2$ die unter der Formel I angegebene Bedeutung haben und der Ring A nicht weiter substituiert oder durch Niederalkyl, insbesondere Methyl,

Niederalkoxy, insbesondere Methoxy, Halogen bis Atomnummer 35 oder Cyano einfach weitersubstituiert ist, und deren Säureadditionssalze. In erster Linie betrifft die Erfindung Verbindungen der allgemeinen Formel I, in denen $R_1$ und $R_2$ je Methyl oder zusammen Tetramethylen bedeuten, $R_3$ Niederalkyl, insbesondere Methyl, oder Niederalkinyl, insbesondere 2-Propinyl, oder vor allem Wasserstoff bedeutet und $Y_1$ und $Y_2$ zusammen eine zusätzliche Bindung oder vorzugsweise je ein Wasserstoffatom bedeuten, Ring A nicht weiter substituiert oder durch Methyl, Methoxy, Halogen bis Atomnummer 35 oder Cyano einfach weitersubstituiert ist, und der stickstoffhaltige Ring vorzugsweise in 5- oder 6-Stellung und der gegebenenfalls vorhandene Substituent vorzugsweise in 7-Stellung des Benzofuranringsystems steht, und deren Säureadditionssalze, wie z.B. das 4-(2,3-Dimethyl-5-benzofuranyl)-piperidin, das 4-(2,3-Dimethyl-6-benzofuranyl)-piperidin und das 4-(6,7,8,9-Tetrahydro-2-dibenzofuranyl)-piperidin und deren Säureadditionssalze, wobei als solche Salze in allen Fällen pharmazeutisch annehmbare bevorzugt werden.

Die neuen Tetrahydropyridin- und Piperidinderivate der allgemeinen Formel I und ihre Säureadditionssalze können nach an sich bekannten Methoden hergestellt werden, beispielsweise indem man

a) in einer Verbindung der allgemeinen Formel II

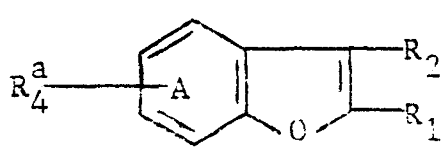

(II)

in welcher $R_4^a$ einen 1,2,3,6-Tetrahydro-4-pyridyl- oder 4-Piperidylrest bedeutet, welcher gegebenenfalls an mindestens einem der dem Stickstoffatom benachbarten Ringkohlenstoffatome durch einen durch zwei Wasserstoffatome ersetzbaren, zweiwertigen Rest $X_a$ substituiert ist und an

dessen Stickstoffatom ein definitionsgemässer Rest $R_3$, der gegebenenfalls in seiner 1-Stellung durch einen definitionsgemässen Rest $X_a$ substituiert ist, oder ein einen definitionsgemässen Rest $X_a$ und Niederalkoxy tragendes Kohlenstoffatom gebunden ist, wobei insgesamt mindestens ein definitionsgemässer Rest $X_a$ vorhanden sein muss, $R_1$ und $R_2$ die unter der Formel I angegebene Bedeutung haben und der Ring A nicht weiter substituiert oder weiter substituiert ist, den oder die Reste $X_a$ und, falls vorhanden, das vorgenannte Niederalkoxy durch Wasserstoff ersetzt, oder

b) in einer Verbindung der allgemeinen Formel III

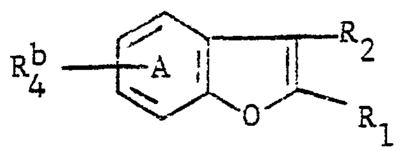

(III)

in welcher $R_4^b$ einen am gegebenenfalls quaternären Stickstoffatom den Rest $R_3$ tragenden, gegegebenenfalls partiell hydrierten 4-Pyridylrest bedeutet, und $R_1$ und $R_2$ die unter der Formel I angegebene Bedeutung haben und der Ring A nicht weiter substituiert oder weiter substituiert ist, den Rest $R_4^b$ zu einem in 1-Stellung durch $R_3$ substituierten 1,2,3,6-Tetrahydro-4-pyridyl- oder 4-Piperidylrest reduziert, oder

c) in einer Verbindung der allgemeinen Formel IV

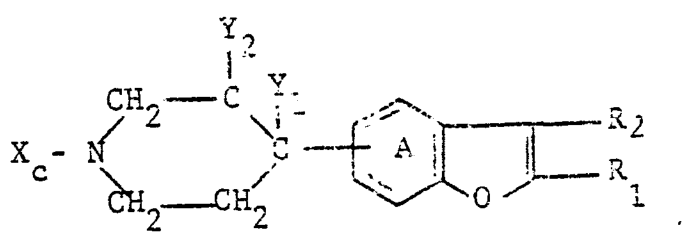

(IV)

in welcher $X_c$ einen durch Wasserstoff ersetzbaren Rest bedeutet und $R_1$, $R_2$, $Y_1$ und $Y_2$ die unter der Formel I angegebene Bedeutung haben und der Ring A nicht weiter

substituiert oder weiter substituiert ist, den Rest $X_c$ durch Wasserstoff ersetzt, oder

d) aus einer Verbindung der allgemeinen Formel V

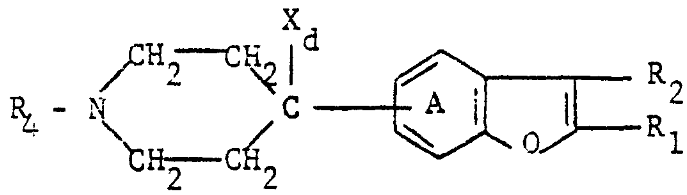

(V)

in welcher $X_d$ gegebenenfalls verestertes Hydroxy bedeutet und $R_1$, $R_2$ und $R_3$ die unter der Formel I angegebene Bedeutung haben und der Ring A nicht weiter substituiert oder weiter substituiert ist, die Verbindung der Formel

$$H - X_d \qquad \text{(VI)}$$

abspaltet, oder

e) in einer Verbindung der allgemeinen Formel VII

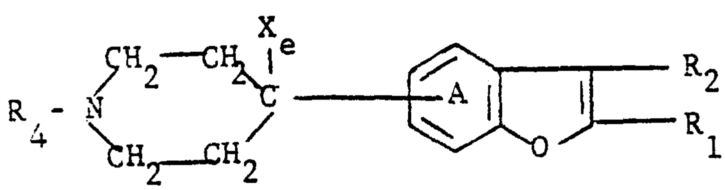

(VII)

in welcher $X_e$ einen durch Wasserstoff ersetzbaren Rest bedeutet und $R_1$, $R_2$ und $R_3$ die unter der Formel I angegebene Bedeutung haben, und der Ring A nicht weiter substituiert oder weiter substituiert ist, den Rest $X_e$ durch Wasserstoff ersetzt, oder

f) eine Verbindung der allgemeinen Formel VIII

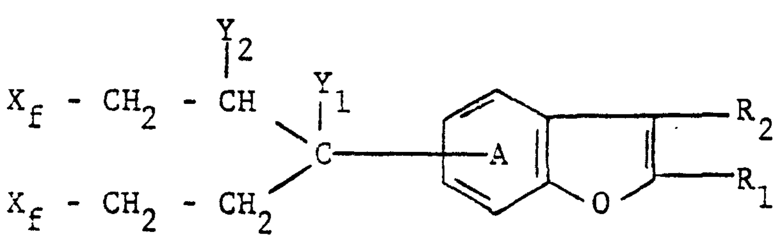

(VIII)

in welcher eines der Symbole $X_f$ die Gruppe $-NHR_3$, in welcher $R_3$ die unter der Formel I angegebene Bedeutung hat, und das andere eine reaktionsfähige veresterte Hydroxygruppe bedeutet, und $R_1$, $R_2$, $Y_1$ und $Y_2$ die unter der Formel I angegebene Bedeutung haben, und der Ring A nicht weiter substituiert oder weiter substituiert ist, cyclisiert, oder

g) eine Verbindung der allgemeinen Formel IX

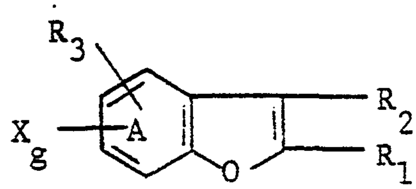

(IX)

und eine Verbindung der allgemeinen Formel X

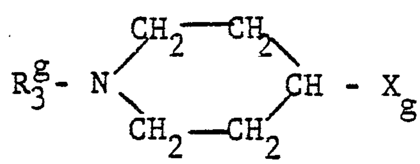

(X)

worin eines der Symbole $X_g$ einen Halogenomagnesiumrest oder einen Alkalimetallrest und das andere Halogen bedeutet, $R_3^g$ einen Rest entsprechend der unter der Formel I angegebenen Definition für $R_3$ mit Ausnahme von Wasserstoff bedeutet und $R_1$ und $R_2$ die unter der Formel I angegebene Bedeutung haben, und der Ring A nicht weiter substituiert oder weiter substituiert ist, miteinander umsetzt, oder

h) auf das Gemisch aus einer Verbindung der allgemeinen Formel XI

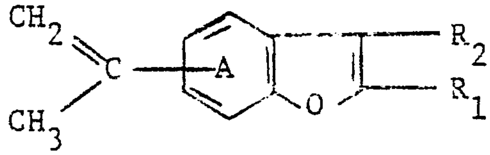

(XI)

in welcher $R_1$ und $R_2$ die unter der Formel I angegebene Bedeutung haben und der Ring A nicht weiter substituiert oder weiter substituiert ist, einer Verbindung der allgemeinen Formel XII,

$$R_3 - NH_2 \qquad (XII)$$

in welcher $R_3$ die unter der Formel I angegebene Bedeutung hat, und der mindestens doppeltmolaren Menge Formaldehyd, oder auf das aus den vorgenannten drei Reaktionskomponenten erhältliche rohe Reaktionsprodukt eine Protonensäure einwirken lässt, und/oder gewünschtenfalls in eine Verbindung der allgemeinen Formel I, in welcher $R_3$ Wasserstoff bedeutet und $R_1$, $R_2$, $Y_1$ und $Y_2$ die unter der Formel I angegebene Bedeutung haben, einen von Wasserstoff verschiedenen Rest $R_3$ einführt, und/oder gewünschtenfalls eine Verbindung der allgemeinen Formel I, in welcher der Ring A durch Brom substituiert und der Rest $R_3$ von Wasserstoff verschieden ist, während $R_1$, $R_2$, $Y_1$ und $Y_2$ die unter der Formel I angegebene Bedeutung haben, oder eine entsprechende Verbindung mit Jod anstelle von Brom, mit einem Metallcyanid umsetzt, und/oder gewünschtenfalls eine Verbindung der allgemeinen Formel I, in welcher der Ring A durch Niederalkoxy substituiert ist, während $R_1$, $R_2$, $R_3$, $Y_1$ und $Y_2$ die unter der Formel I angegebene Bedeutung haben, oder eine entsprechende Verbindung mit gegebenenfalls substituiertem Benzyloxy anstelle von Niederalkoxy, mit einem ätherspaltendem Mittel behandelt, und/oder gewünschtenfalls eine Verbindung der allgemeinen Formel I in anderer an sich bekannter Weise in eine andere Verbindung der allgemeinen Formel I umwandelt, und/oder ein erhältliches Isomerengemisch (Racematgemisch) in die reinen

Isomeren (Racemate) auftrennt, und/oder ein erhältliches Racemat in die optischen Antipoden aufspaltet und/oder eine erhältliche freie Verbindung in ein Salz, oder ein erhaltenes Salz in die freie Verbindung oder in ein anderes Salz überführt.

In den Ausgangsstoffen der allgemeinen Formel II sind die durch zwei Wasserstoffatome ersetzbaren Reste $X_a$ beispielsweise Oxo- oder Thioxoreste. Der Rest $R_4^a$ entspricht in diesem Falle vorzugsweise der Teilformel IIa

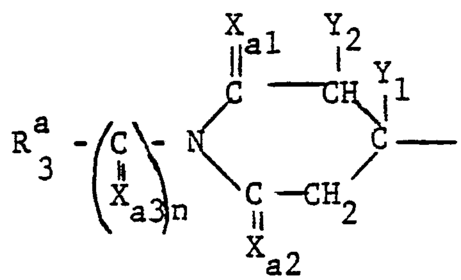

(IIa)

in welcher mindestens eines der Symbole $X_{a1}$, $X_{a2}$ und $X_{a3}$ einen Oxo- oder Thioxorest, und die bzw. das übrigbleibende dieser Symbole je zwei Wasserstoffatome bedeuten, $R_3^a$ für Wasserstoff, einen gegebenenfalls um eine Methylengruppe verminderten Rest entsprechend der Definition für $R_3$, oder falls n eins und $X_{a3}$ Oxo ist, Niederalkoxy und somit zusammen mit $CX_{a3}$ Niederalkoxycarbonyl, und n null oder eins bedeutet und $Y_1$ und $Y_2$ die unter der Formel I angegebene Bedeutung haben.

In Ausgangsstoffen der allgemeinen Formel II, in denen Reste $X_a$ Oxoreste sind und/oder in denen sich am Ringstickstoffatom Niederalkoxycarbonyl befindet, können in an sich bekannter Weise die Oxoreste durch Wasserstoff ersetzt und/oder Niederalkoxycarbonyl zu Methyl reduziert werden, z.B. durch Reduktion mit Hydridreduktionsmitteln, z.B. mit Alkalimetall-Erdmetallhydriden, wie Lithiumaluminiumhydrid, Natrium-bis-(2-methoxyäthoxy)-aluminiumhydrid oder Natrium-tris-(2-methoxyäthoxy)-aluminiumhydrid, oder Boran oder Diboran, in einem ätherartigen Lösungsmittel,

0006524

wie Diäthyläther, Tetrahydrofuran, Dibutyläther oder Diäthylenglykoldiäthyläther oder deren Gemischen, beispielsweise bei Temperaturen zwischen ca. 0° und 100°C bzw. Siedetemperatur des verwendeten Lösungsmittels, falls diese unter 100°C liegt, und vorzugsweise bei etwa 20°C bis etwa 65°C.

Der Ersatz von Thioxoresten $X_a$ durch Wasserstoff kann ebenfalls in an sich bekannter Weise erfolgen, beispielsweise durch Umsetzung von entsprechenden Ausgangsstoffen der allgemeinen Formel II mit Raney-Nickel, vorzugsweise in Anwesenheit geeigneter inerten organischer Lösungs- und/oder Verdünnungsmittel, wie Aethanol, Methanol oder Aceton, z.B. bei Temperaturen zwischen 0° und Siedetemperatur des Reaktionsmediums, vorzugsweise bei Raumtemperatur bis etwa 80°C bzw. Siedetemperatur des Reaktionsmediums.

In den Ausgangsstoffen der allgemeinen Formel III ist der Rest $R_4^b$ beispielsweise ein 4-Pyridylrest oder entspricht einer der Teilformeln IIIa oder IIIb

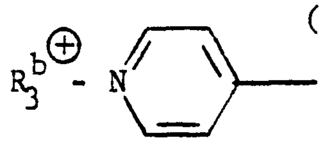
(IIIa)

oder

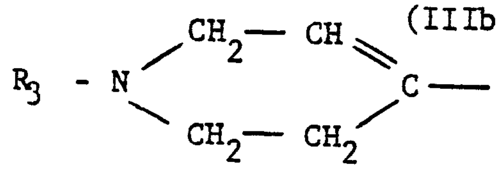
(IIIb)

in welcher $R_3^b$ einen Rest der unter der Formel I gegebenen Definition für $R_3$ mit Ausnahme von Wasserstoff bedeutet und $R_3$ die unter der Formel I angegebene Bedeutung hat. Verbindungen der allgemeinen Formel III mit einem Rest der Teilformel IIIb als $R_4^b$ fallen bereits unter die allgemeine Formel I. Die Reduktion solcher Verbindungen wie derjenigen mit 4-Pyridyl oder einem Rest der Teilformel IIIa als $R_4^b$ zu Verbindungen der allgemeinen Formel I, in welchen $Y_1$ und $Y_2$ Wasserstoff bedeuten, kann beispiels-

weise mittels katalytisch aktiviertem Wasserstoff unter Verwendung von üblichen Hydrierungskatalysatoren, beispielsweise von Edelmetallkatalysatoren wie Palladium auf Kohle oder Platinoxid, von Rhodiumkatalysatoren, wie Rhodium auf Kohle oder auf Aluminiumoxid, oder von Legierungsskelett-Katalysatoren, wie Raney-Nickel, in einem inerten organischen Lösungsmittel, wie Methanol, Aethanol oder Dioxan, bei Raumtemperatur und Normaldruck oder mässig erhöhten Temperaturen bis ca. 100°C und erhöhten Drücken bis ca. 100 bar durchgeführt werden.

Ausgangsstoffe der allgemeinen Formel III, in denen $R_4^b$ ein Rest der Teilformel IIIa ist, kann man auch in an sich bekannter Weise zu den entsprechenden, unter die allgemeine Formel I fallenden Verbindungen mit einem Rest der Teilformel IIIb als Rest $R_4^b$ reduzieren. Diese Reduktion wird vorzugsweise mit Hilfe von Natrium- oder Kaliumborhydrid in organisch-wässrigem Medium durchgeführt, indem man beispielsweise zur vorgelegten Lösung des entsprechenden Ausgangsstoffes der allgemeinen Formel III in einem organischen, mit Wasser mischbaren Lösungsmittel, z.B. in einem niederen Alkanol wie Methanol oder Aethanol oder deren Gemischen mit Wasser allmählich eine wässrige Lösung von Natriumborhydrid zufügt und das Reaktionsgemisch anschliessend noch einige Zeit weiterreagieren lässt, wobei eine Reaktionstemperatur zwischen ca. 5 und 60°C, vorzugsweise Raumtemperatur bis 35°C, eingehalten wird. Die Herstellung der Ausgangsstoffe der allgemeinen Formel III wird weiter unten erläutert.

In den Ausgangsstoffen der allgemeinen Formel IV für das Verfahren c) sind durch Wasserstoff ersetzbare Reste $X_c$ z.B. Reste, die mittels Solvolyse, insbesondere Hydrolyse oder Amino- bzw. Ammonolyse, oder mittels Reduktion durch Wasserstoff ersetzt werden können.

Mittels Solvolyse durch Wasserstoff ersetzbare Reste sind beispielsweise Acylreste, wie Acylreste von

organischen Säuren, z.B. gegebenenfalls halogenierte, wie fluorierte, niedere Alkanoylreste, beispielsweise Butyryl, Propionyl, Acetyl oder Trifluoracetyl, oder Benzoylreste, oder gegebenenfalls funktionell abgewandelte Carboxylgruppen, z.B. veresterte Carboxylgruppen, wie Alkoxycarbonylreste, z.B. der tert.-Butoxycarbonylrest oder der Methoxycarbonylrest, Aralkoxycarbonylreste, wie Phenylniederalkoxycarbonylreste, z.B. Carbobenzoxy, weiter Cyanoreste oder Halogencarbonylreste, z.B. der Chlorcarbonylrest, β-Arylsulfonyläthoxycarbonylreste, wie β-Toluolsulfonyl- oder β-Brombenzolsulfonyläthoxycarbonyl, oder β-Arylthioäthyl- oder β-Arylsulfonyläthyl- oder β-Arylsulfonyläthylreste, wie β-(p-Toluolsulfonyl)-äthyl- oder 2-(p-Tolylthio)-äthylreste, oder auch Silylreste, wie der Trimethylsilylrest.

Durch Reduktion abspaltbare Reste sind beispielsweise α-Arylalkylreste, wie Benzylreste, oder α-Aralkoxycarbonylreste, wie Benzyloxycarbonylreste, Arylsulfonyl-, z.B. p-Toluolsulfonylreste, oder 2-Halogen-alkoxycarbonylreste, wie der 2,2,2-Trichloräthoxy-, 2-Jodäthoxy- oder 2,2,2-Tribromäthoxy-carbonylrest.

Die Hydrolyse von Verbindungen der allgemeinen Formel IV kann in alkalischem oder saurem Medium durchgeführt werden. Beispielsweise wird sie durch längeres Erhitzen mit einem Alkalihydroxid, insbesondere Natrium- oder Kaliumhydroxid, in einer Hydroxyverbindung in Gegenwart von wenig Wasser bei Temperaturen zwischen ca. 80° und 200°C vollzogen. Als Reaktionsmedium eignet sich beispielsweise Aethylenglykol oder ein niederer Monoalkyläther desselben, ferner bei Durchführung der Hydrolyse im geschlossenen Gefäss auch ein niederes Alkanol wie Methanol, Aethanol oder Butanol. Ferner lassen sich insbesondere Verbindungen der allgemeinen Formel IV, in denen der Rest $X_c$ eine Cyanogruppe, d.h. den Acylrest der Cyansäure, oder eine Chlorcarbonylgruppe bedeutet, auch durch Erhitzen

mit einer Mineralsäure in einem organisch-wässrigen oder wässrigen Medium, z.B. durch mehrstündiges Kochen in einem Gemisch von 85%-iger Phosphorsäure und Ameisensäure, oder durch mehrstündiges Erwärmen in 48%-iger Bromwasserstoffsäure (oder in Bromwasserstoffsäure) oder in Bromwasserstoffsäure-Essigsäure-Gemisch auf ca. 60-100°C, vorzugsweise 60-70°C hydrolysieren.

Weitere abspaltbare Gruppen Xc sind die durch Addition einer anstelle von Xc befindlichen Methylgruppe an Azodicarbonsäure-diniederalkylester entstehenden Gruppen, die vorzugsweise durch Hydrolyse in saurem Medium, insbesondere Kochen in verdünnter, z.B. 1-n. Salzsäure, unter Freisetzung von Hydrazodicarbonsäure-diniederalkylester abgespalten werden.

Ein durch Solvolyse abspaltbarer Rest ist z.B. der Tert.butoxycarbonylrest, der unter wasserfreien Bedingungen durch Behandeln mit einer geeigneten Säure, wie Trifluoressigsäure, abgespalten werden kann.

Die Aminolyse bzw. Ammonolyse kann in üblicher Weise erfolgen, beispielsweise durch Umsetzung mit Ammoniak oder einem Amin, wie Hydrazin oder einem Mono- oder Dialkylamin oder Alkylen- oder Oxa-, Aza- oder Thiaalkylenamin, z.B. mit Ammoniak, Hydrazin, Methyl- oder Dimethylamin, Morpholin oder Piperidin, erforderlichenfalls in einem inerten Lösungsmittel und/oder bei erhöhter Temperatur.

Mittels Reduktion durch Wasserstoff ersetzbare Reste $X_c$ sind beispielsweise α-Arylalkylreste, wie der Benzylrest, oder α-Aralkoxycarbonylreste, wie der Benzyloxycarbonylrest, die in üblicher Weise mittels Hydrogenolyse durch Wasserstoff ersetzt werden können, z.B. mittels Wasserstoff in Gegenwart eines Hydrierungskatalysators, beispielsweise Platin, Palladium oder Raney-Nickel, und gegebenenfalls von Chlorwasserstoff, bei Raumtemperatur und Normaldruck oder bei mässig erhöhten Temperaturen und Drücken in gee...

ten organischen Lösungsmitteln, wie z.B. Methanol, Aethanol oder Dioxan. Weitere mittels Reduktion durch Wasserstoff ersetzbare Reste $X_c$ sind beispielsweise 2-Halogen-alkoxycarbonylreste, wie der 2,2,2-Trichloräthoxycarbonylrest oder der 2-Jodäthoxy- oder 2,2,2-Tribromäthoxycarbonylrest, die in üblicher Weise, insbesondere durch metallische Rekuktion (sog. naszierenden Wasserstoff) entfernt werden können. Naszierender Wasserstoff kann dabei durch Einwirkung von Metall oder Metall-Legierungen, wie Amalgamen, auf Wasserstoff liefernde Mittel, wie Carbonsäuren, Alkohole oder Wasser erhalten werden, wobei insbesondere Zink oder Zinklegierungen zusammen mit Essigsäure in Betracht kommen. Die Reduktion von 2-Halogen-alkoxy-carbonylresten kann ferner durch Chrom(II)-verbindungen, wie Chrom(II)-chlorid oder Chrom(II)-acetat erfolgen.

Ein durch Reduktion abspaltbarer Rest $X_c$ kann auch eine Sulfonylgruppe, wie eine Niederalkansulfonylgruppe oder Arylsulfonylgruppe, z.B. Methansulfonyl oder p-Toluolsulfonyl, sein, die in üblicher Weise durch Reduktion mit naszierendem Wasserstoff, z.B. durch ein Alkalimetall, wie Lithium oder Natrium in flüssigem Ammoniak, oder auch elektrolytisch abgespalten werden kann.

Wenn in den Ausgangsstoffen der allgemeinen Formel V $X_d$ freies Hydroxy ist, ist das Verfahren d) eine Wasserabspaltung, die z.B. durch Erwärmen der entsprechenden Ausgangsstoffe, mit Vorteil unter Abscheidung des gebildeten Wassers und vorzugsweise in Gegenwart einer starken Säure, z.B. von konz. Salzsäure in Eisessig oder von Schwefelsäure, die konzentriert, aber in geringen Mengen verwendet wird, oder von p-Toluolsulfonsäure erfolgen kann. Die Wasserabspaltung kann auch durch Erhitzen in einem inerten organischen Lösungsmittel vorgenommen werden, z.B. in einem mit Wasser nicht mischbaren Lösungsmittel, wie Toluol oder Xylol, bei deren Siedetemperatur und mit Vorteil unter Wasserabscheidung, oder in Hexameth-

ylphosphorsäuretriamid, z.B. auf Temperaturen zwischen 180 und 240°C.

Als weitere Wasserabspaltungsmittel eignen sich z.B. anorganische Säureanhydride wie Phosphorpentoxid oder Boroxid, gewisse organische Säuren wie Ameisensäure und Oxalsäure, saure Salze starker Säuren, wie Kaliumbisulfat, oder Ionenaustauscherharze, z.B. Kondensationsprodukte von Phenolsulfonsäuren und Formaldehyd.

Ist in den Ausgangsstoffen der allgemeinen Formel V $X_d$ verestertes Hydroxy, z.B. Acetoxy, Benzoyloxy, Methansulfonyloxy oder Halogen, insbesondere Chlor oder Brom, so wird als Verbindung der allgemeinen Formel VI eine Säure abgespalten. Dies kann ebenfalls durch blosses Erhitzen erfolgen, wobei gegebenenfalls als Reaktionsprodukt das entsprechende Säureadditionssalz der gewünschten Verbindung der allgemeinen Formel I erhalten wird. Gegebenenfalls erfolgt die Säureabspaltung in alkalischem oder saurem Medium in der Wärme, z.B. mit Alkalihydroxiden in wässrigem oder niederalkanolischem Medium, oder mit Stickstoffbasen wie Piperidin, Pyridin, Lutidin, Collidin oder Chinolin, bzw. mit Polyphosphorsäure. Das veresterte Hydroxy $X_d$ kann auch <u>in situ</u> aus freiem Hydroxy gebildet werden, d.h. man kann Verbindungen mit freiem Hydroxy als $X_d$ mit geeigneten Acylierungsmitteln, z.B. anorganischen Säurehalogeniden wie Phosphortrichlorid, Phosphoroxychlorid oder Thionylchlorid in geeigneten inerten organischen Lösungsmitteln, wie z.B. Chloroform, oder organischen Säureanhydriden und -halogeniden, wie Acetanhydrid, Phthalsäureanhydrid, Acetylchlorid oder Acetylbromid, in Anwesenheit oder vorzugsweise Abwesenheit inerter organischer Lösungs- oder Verdünnungsmittel bei mässig erhöhter bis Siedetemperatur umsetzen, wobei direkt Verbindungen der allgemeinen Formel I, in denen $Y_1$ und $Y_2$ zusammen eine zusätzliche Bindung bedeuten, entstehen, während man unter

milderen Bedingungen mit organischen Säureanhydriden oder -halogeniden Ausgangsstoffe der allgemeinen Formel V erhalten kann, in denen $X_d$ ein entsprechender Acyloxyrest ist.

In den Ausgangsstoffen der allgemeinen Formel VII für das Verfahren e) ist der Rest $X_e$ beispielsweise eine Carboxygruppe, die in üblicher Weise, insbesondere durch Erhitzen auf Temperaturen von etwa 220 bis etwa 280°C, oder auch höher, gegebenenfalls in einem Inertgas-, z.B. Stickstoffstrom, durch Wasserstoff ersetzt werden kann. Gegebenenfalls wird die Decarboxylierung in Gegenwart eines Erdalkalimetallhydroxids, insbesondere Calciumoxid, durchgeführt; man kann aber auch eine von der allgemeinen Formel VII umfasste Carbonsäure zunächst in ein Alkalimetall- oder Erdalkalimetallsalz, oder z.B. auch in ein Kupfer-, Quecksilber- oder Silbersalz überführen und dieses Salz erhitzen, z.B. auf die obengenannten Temperaturen.

Weitere Reste $X_e$ sind z.B. Hydroxy und veräthertes Hydroxy, wie Niederalkoxy, vor allem Methoxy oder Aethoxy, die sich infolge ihrer Benzylstellung mittels Hydrogenolyse durch Wasserstoff ersetzen lassen. Die Hydrogenolyse kann in üblicher Weise, z.B. unter Verwendung der zum Verfahren b) genannten Katalysatoren und Lösungsmittel, durchgeführt werden.

In den Ausgangsstoffen der allgemeinen Formel VIII für das Verfahren f) ist eine reaktionsfähige veresterte Hydroxygruppe $X_f$ beispielsweise eine mit einer starken Säure, z.B. einer Mineralsäure, wie einer Halogenwasserstoffsäure, z.B. mit Jod-, Brom- oder Chlorwasserstoffsäure, oder mit einer organischen Sulfonsäure, z.B. mit Benzol-, p-Toluol-, p-Brombenzol-, Methan- oder Aethansulfonsäure veresterte Hydroxygruppe.

Die unter Abspaltung von $HX_f$ erfolgende Cyclisierung kann in üblicher Weise, beispielsweise durch Erwärmen oder mässiges Erhitzen, z.B. bis auf 200°, trocken oder erforderlichenfalls in Gegenwart eines inerten Lösungsmittels und/oder in Gegenwart eines Kondensationsmittels bewirkt werden. Ausgehend von Verbindungen, in denen $X_f$ reaktionsfähiges verestertes Hydroxy ist, verwendet man z.B. ein basisches Kondensationsmittel, wie ein tertiäres Amin, z.B. Triäthylamin oder Pyridin, oder eine anorganische Base, z.B. ein Alkali- oder Erdalkalimetallcarbonat oder -hydroxid, wie Kaliumhydroxid.

Die ihrerseits neuen Ausgangsstoffe der allgemeinen Formel VIII können in an sich bekannter Weise, z.B. durch Umsetzung von entsprechenden Verbindungen, in denen beide Symbole $X_f$ reaktionsfähige veresterte Hydroxygruppen bedeuten und deren Herstellung weiter unten erläutert ist, mit Verbindungen der weiter oben angegebenen allgemeinen Formel XII, d.h. mit Niederalkyl-, Niederalkenyl-, Niederalkinyl-, Cycloalkyl oder Cycloalkylniederalkylaminen, hergestellt werden. Die Herstellung der Verbindungen der allgemeinen Formel VIII kann gewünschtenfalls in situ, d.h. im gleichen Arbeitsgang und unter Verwendung der gleichen Lösungsmittel und/oder Kondensationsmittel wie bei der anschliessenden Cyclisierung, durchgeführt werden.

Der in einer der beiden Reaktionskomponenten der allgemeinen Formeln IX und X für das Verfahren g) befindliche Halogenmagnesium- oder Alkalimetall-rest $X_g$ ist beispielsweise ein Chlormagnesium-, Brommagnesium-, Jodmagnesium- bzw. Lithium-rest und das in der andern Reaktionskomponente befindliche Halogen $X_g$ z.B. Chlor, Brom oder Jod. Die Umsetzungen gemäss Verfahren f) können in üblicher Weise erfolgen, beispielsweise Umsetzungen mit Halogenmagnesiumverbindungen IX oder X in einem inerten Lösungsmittel, wie einem aliphatischen Aether, z.B. in Diäthyläther, Tetrahydrofuran oder Dioxan, und solche mit Lithiumverbindungen IX oder X z.B. in einem Kohlenwasserstoff, wie Hexan, Benzol oder Toluol, erforderlichenfalls in Gegenwart eines katalytischen Mittels, wie eines

Schwermetallsalzes, z.B. eines Halogenides, wie des Chlorides des Kupfers, und/oder bei erhöhter Temperatur, z.B. bei Siedetemperatur des Reaktionsmediums.

Diejenigen Reaktionskomponenten der allgemeinen Formel IX oder X, in denen $X_g$ ein Alkalimetallrest oder ein Halogenmagnesiumrest bedeutet, werden vorzugsweise in situ, beispielsweise durch übliche Umsetzung der entsprechenden ebenfalls unter die allgemeine Formel IX bzw. X fallenden Halogenverbindungen mit einer reaktionsfähigen Alkalimetallverbindung, z.B. Butyllithium, bzw. mit Magnesium, vorteilhaft in feinverteilter Form in einem inerten Lösungsmittel, wie einem aliphatischen Aether, z.B. einem der vorgenannten, hergestellt und, vorteilhaft ohne Isolierung, weiter umgesetzt.

Einige Ausgangsstoffe der allgemeinen Formel IX und X, in welchen $X_g$ Halogen bedeutet, sind bekannt und weitere analog zu den bekannten Verbindungen herstellbar.

Beim Verfahren h) wird der Formaldehyd z.B. in oligomerer Form, wie als Paraformaldehyd, oder als wässrige, z.B. 30-35%-ige, Lösung eingesetzt. Als Protonensäure wird vorzugsweise eine Mineralsäure, wie Salzsäure, insbesondere konzentrierte Salzsäure, oder Schwefelsäure, insbesondere mässig verdünnte, wie 66%-ige, Schwefelsäure, verwendet. Bezogen auf die Verbindungen der allgemeinen Formel XII werden die Säuren im Ueberschuss, d.h. in einer Menge von mehr als einem Aequivalent, vorzugsweise mehr als zwei Aequivalenten, eingesetzt. Die Reaktionstemperatur liegt, nach anfänglichem Kühlen zur Kontrolle der exothermen Reaktion, vorzugsweise zwischen 60 und 110°C, insbesondere bei 90-100°C, unter Einhaltung einer Reaktionsdauer von beispielsweise etwa 1 bis 10 Stunden.

Eine gegebenenfalls vorteilhaftere Variante des Verfahrens h) besteht darin, dass man zunächst eine Verbindung der allgemeinen Formel XI mit der doppeltmolaren Menge Formaldehyd, beispielsweise als ca. 35%-ige wässrige Lösung, und der äquimolaren Menge eines mineralsauren Salzes, insbesondere des Hydrochlorids, einer Verbindung der allgemeinen Formel XII, oder mit der entsprechenden Menge einer freien Verbindung der allgemeinen Formel XII und der äquinormalen Menge Mineralsäure umsetzt und anschliessend das entstandene rohe Reaktionsprodukt in den gewünschten Endstoff umwandelt, indem man unter Kühlen, z.B. bei 50-70°C allmählich weitere Mineralsäure, wie konz. Schwefelsäure, zufügt und die Reaktion durch Erhitzen auf z.B. 90-95°C zu Ende führt.

In Verbindungen der allgemeinen Formel I, worin $R_3$ Wasserstoff ist, kann man gewünschtenfalls in üblicher Weise, beispielsweise durch Umsetzung mit einem reaktionsfähigen Ester, vorzugsweise einem Halogen- oder Sulfonsäureester, z.B. dem Chlor-, Brom- oder Jodwasserstoffsäureester oder Benzol-, p-Toluol-, p-Brombenzol- oder Methansulfonsäureester eines Niederalkanols, Niederalkenols, Niederalkinols oder Cycloalkylniederalkanols einen von Wasserstoff verschiedenen Rest $R_3$, oder durch Umsetzung mit einem Niederalkanal oder Diniederalkylketon unter reduzierenden Bedingungen, beispielsweise in Gegenwart von durch Edelmetallkatalysatoren wie Palladium, z.B. auf Kohle, oder Platin, oder von Raney-Nickel, katalytisch aktiviertem Wasserstoff, Niederalkyl $R_3$ einführen, erforderlichenfalls in einem inerten Lösungsmittel und/oder bei erhöhtem Druck und/oder erhöhter Temperatur. Analog kann auch ein Cycloalkylniederalkylrest $R_3$ eingeführt werden. Ebenfalls möglich, aber weniger vorteilhaft als die direkte Herstellung entsprechender Verbindungen der allgemeinen Formel I z.B. nach Verfahren a), ist die analoge Einführung eines Cycloalkylrestes $R_3$.

Zur Umsetzung einer Verbindung der allgemeinen Formel I, in der Ring A durch Brom substituiert und $R_3$ von Wasserstoff verschieden ist, oder einer analogen Verbindung mit Jod anstelle von Brom, verwendet man als Metallcyanid beispielsweise Kupfer(I)-cyanid und führt die Reaktion bei erhöhter Temperatur, z.B. zwischen etwa 130°C und etwa 210°C, in einem inerten organischen Lösungsmittel mit entsprechender Siede- oder Zersetzungstemperatur, vorzugsweise in einem Amid oder Lactam, wie Dimethylformamid - bei Temperaturen bis 140°C - bzw. 1-Methyl-2-pyrolidinon, oder in Sulfolan (Tetrahydrothiophen-1,1-dioxid) durch. Ausgangsstoffe mit Jod anstelle von Brom im Ring A können analog zu den Verbindungen der allgemeinen Formel I mit Brom als Substituent des Ringes A unter Verwendung von entsprechenden Ausgangsstoffen bzw. Vorprodukten zu solchen hergestellt werden.

Als ätherspaltende Mittel zur Umwandlung von Verbindungen der allgemeinen Formel I, in denen der Ring A durch Niederalkoxy, insbesondere Methoxy, substituiert ist, in entsprechende Verbindungen mit Hydroxy in gleicher Stellung eignen sich z.B. Alkalicyanide, wie Natriumcyanid, in oxidierten Dialkylsulfiden oder ihren cyclischen Analogen, insbesondere in Dimethylsulfoxid bzw. Sulfolan, in der Wärme, z.B. zwischen ca. 150 bis 200°C. Die Umwandlung von entsprechenden Verbindungen mit gegebenenfalls substituiertem Benzyloxy, z.B. Benzyloxy oder p-Niederalkoxybenzyloxy, wie p-Methoxybenzyloxy als Substituent des Ringes A in entsprechende Verbindungen mit Hydroxy in gleicher Stellung kann in analoger Weise, jedoch auch unter milderen Bedingungen, z.B. durch Behandlung mit konz. Halogenwasserstoffsäuren, gegebenenfalls in Gegenwart von Essigsäure in der Wärme, z.B. bei Temperaturen zwischen ca. 70°C und Siedetemperatur des Reaktionsgemisches, oder als Hydrogenolyse durch Einwirkung von Wasserstoff in Gegenwart eines Hydrierungskatalysators, beispielsweise Platin, Palladium oder Raney- Nickel, und gegebenenfalls von Chlorwasserstoff, bei Raumtemperatur und Normaldruck oder bei mässig erhöhten Temperaturen und Drücken in geeigneten organischen Lösungsmitteln, wie z.B. Methanol, Aethanol oder Dioxan erfolgen. Die Aetherspaltung, insbesondere diejenige von Benzyläthern, und vor allem der Hydrogenolyse der letzteren kann auch zusammen mit der Abspaltung eines entsprechenden Restes

$X_c$ gemäss Verfahren c) erfolgen. Ausgangsstoffe mit einer gegebenenfalls substituierten Benzyloxygruppe als Substituent des Ringes A können z.B. analog zu Verbindungen der allgemeinen Formel I mit Niederalkoxy in gleicher Stellung des Ringes A unter Verwendung von entsprechenden Ausgangsstoffen bzw. Vorprodukten zu solchen hergestellt werden. Man kann aber auch in den in der nachstehend beschriebenen Reaktionsfolge zur Herstellung von Ausgangsstoffen der allgemeinen Formel II vorkommenden, im Ring A durch Methoxy substituierten 4-, 5-, 6- oder 7-Benzofurancarbonsäuren die Methoxygruppe, wie vorstehend angegeben, spalten und, gegebenenfalls nach Bildung entsprechender Niederalkylester, die Hydroxygruppe in bekannter Weise in die Benzyloxygruppe überführen und die erhaltenen Carbonsäuren bzw. Niederalkylester analog zur unten angegebenen Reaktionsfolge weiterbehandeln, d.h. zunächst zu entsprechenden Benzyloxy-benzofuranmethanolen reduzieren.

Ausgangsstoffe der allgemeinen Formel II, in denen $X_{a1}$ und/oder $X_{a2}$ des Restes $R_3^a$ der Teilformel IIa durch zwei Wasserstoffatome ersetzbare Reste bedeuten, insbesondere 2-Piperidinone bzw. Glutarimide, können beispielsweise ausgehend von 4-, 5-, 6- oder 7-Benzofurancarbonsäuren oder deren Niederalkylestern hergestellt werden. Von diesen Verbindungen sind einige Vertreter bekannt, z.B. wurden die 2,3-Dimethyl-6-benzofurancarbonsäure, 2,3,7-Trimethyl-6-benzofurancarbonsäure, 2,3,5-Trimethyl-6-benzofurancarbonsäure, 2,3-Dimethyl-4-benzofurancarbonsäure, 2,3,7-Trimethyl-4-benzofurancarbonsäure und 2,3-Dimethyl-5-benzofurancarbonsäure sowie Niederalkylester derselben von Y. Kawase und M. Takashima im Bull. Chem. Soc. Jap. 40, 1224-1231 (1967) beschrieben Weitere sind analog zu den bekannten Verbindungen, beispielsweise wie die vorgenannten, stets in 2- und 3-Stellung substituierten Carbonsäuren aus entsprechend substituierten Benzofuranen durch Acetylierung nach Friedel-Crafts und Behandlung der Acetylverbindungen mit wässriger

Natriumhypobromitlösung, der Dioxan oder Tetrahydrofuran zugefügt ist, bei 45°C erhältlich. Eine weitere bekannte Methode mit verschiedenen Varianten ist beispielsweise die Umsetzung von gegebenenfalls bevorzugte Substituenten des Ringes A tragenden Salicylsäure- oder m- oder p-Hydroxybenzoesäure-niederalkylestern mit α-Halogen-niederalkanalen, -niederalkanonen oder -cycloalkanonen in Gegenwart eines säurebindenden Mittels, wie Kaliumcarbonat, und Cyclisierung der entstandenen Oxoniederalkyl- oder Oxocycloalkyl-äther, z.B. mittels konz. Schwefelsäure in der Kälte. Zur Cyclisierung verwendete Oxoniederalkyläther können aus den Hydroxybenzoesäureniederalkylestern auch in zwei Stufen, d.h. durch Umsetzung der genannten Ester mit 2-Niederalkinylhalogeniden und Hydratisierung der erhaltenen Niederalkinyläther in üblicher Weise, z.B. durch Behandlung mit Quecksilber(II)-sulfat in wässrig- niederalkanolischer Lösung bei schwach erhöhter Temperatur, hergestellt werden.

Ebenfalls geeignet für die Herstellung von solchen Benzofurancarbonsäuren vom gewünschten Typus, in denen mindestens die 2-Stellung, vorzugsweise auch die 3-Stellung substituiert, d.h. $R_1$ bzw. $R_1$ und $R_2$ von Wasserstoff verschieden sind und z.B. zusammen den Tetramethylenrest bedeuten, ist die von H. Gilman et al., J. Am. Chem. Soc. 57, 2095-2099 (1935) für die Herstellung der 6,7,8,9-Tetrahydro-dibenzofuran-4-carbonsäure beschriebene Metallierung des entsprechend substituierten Benzofurans, z.B. mit Phenyllithium in Aether, Umsetzung der Metallverbindung mit Kohlendioxid, und gewünschtenfalls anschliessend Veresterung nach üblichen Methoden.

Die erhaltenen, gegebenenfalls entsprechend den Definitionen für $R_1$ und $R_2$ sowie im Ring A substituierten 4-, 5-, 6- oder 7-Benzofurancarbonsäure-niederalkylester oder die freien Carbonsäuren werden beispielsweise mittels

komplexen Hydriden, wie Lithiumaluminiumhydrid, in ätherartigen Lösungsmitteln, wie Tetrahydrofuran, zu den entsprechenden 4-,5-, 6- oder 7-Benzofuranmethanolen reduziert und letztere durch partielle Oxidation, z.B. mittels Mangandioxid in einem inerten organischen Lösungsmittel, wie Toluol, in die entsprechenden 4- 5-, 6- oder 7-Benzofurancarboxaldehyde übergeführt. Von diesen Aldehyden sind ebenfalls einzelne Vertreter bekannt,z.B. die durch die für Verbindungen mit von Wasserstoff verschiedenen Resten $R_1$ anwendbare Formylierung mittels Dimethylformamid und Phosphoroxychlorid nach Vilsmeier hergestellten Verbindungen 2,3-Dimethyl-4-methoxy-7-benzofurancarboxaldehyd, 2,3-Dimethyl-5-methoxy-6-benzofurancarboxyaldehyd, 2,3-Dimethyl-6-methoxy-5-benzofurancarboxaldehyd und 2,3-Dimethyl-7-methoxy-6-benzofurancarboxaldehyd [vgl. R. Royer et al.,Bull. Soc. Chim. France 1965, 2607-2616], und der durch Cyclisierung von 2-(1-Methyl-2-oxopropoxy)-benzaldehyd erhaltene 2,3-Dimethyl-7-benzofurancarboxaldehyd [vgl. C. Goldenberg et al., Chim. Thérap. 1966, 221-227], ebenso einige durch Decarboxylierung von entsprechenden, gegebenenfalls weiter substituierten Formyl-2-benzofurancarbonsäuren erhältliche Benzofurancarboxaldehyde mit unsubstituierter 2-Stellung, wie z.B. der 5-Benzofurancarboxaldehyd [vgl. C. Goldenberg et al., vorstehend] und der 6-Methoxy-5-benzofurancarboxaldehyd [vgl. P. Quevel und E. Bisagni, Eur. J. Med. Chem. 9, 335-340 (1974)]. Aus den gegebenenfalls substituierten 4-, 5-, 6- oder 7-Benzofurancarboxaldehyden können unter die allgemeine Formel II fallende 2-Piperidinone beispielsweise erhalten werden, indem man zunächst durch Umsetzung mit Diäthoxyphosphonoacetonitril in Gegenwart von Basen, oder durch Kondensation mit Cyanessigsäure in Gegenwart von Piperidin in Eisessig in der Wärme, entsprechend substituierte 4-, 5-, 6- oder 7-Benzofuranacrylonitrile herstellt, an diese in an sich bekannter Weise einen Malonsäurediniederalkylester addiert, dann im Additionsprodukt die Cyanogruppe katalytisch hydriert, z.B. in Gegenwart von Raney-Nickel bei erhöhtem Druck und erhöhter Temperatur in einem Niederalkanol, wobei unter Lactamisierung der entsprechende 4-(4-, 5- 6- oder 7-Benzofuranyl)-2-oxo-nipecotinsäure-niederalkylester entsteht, welchen man in üblicher Weise zur freien Säure hydrolisieren kann, die sich ebenfalls in üblicher Weise, z.B. durch Kochen in Toluol, zum gewünschten 2-Piperidinon der allgemeinen Formel II decarboxylieren lässt. Man kann die vorgenannten 4-substituierten 2-Oxonipecotinsäure-niederalkylester auch analog zu einer von A.P. Krapcho et al., Tetrahedron Letters 1973, 957, beschriebenen Reaktion durch Erhitzen mit Natriumchlorid und Wasser in Dimethylsulfoxid in einer Stufe in die gewünschten 2-Piperidinone überführen.

Zu unter die allgemeine Formel II fallenden 2,6-Piperidindionen kann man ausgehend von den vorgenannten, gegebenenfalls entsprechend der Definition für $R_1$ und $R_2$ sowie im Ring A substituierten 4-, 5-, 6- oder 7-Benzofurancarboxaldehyden gelangen, indem man diese Aldehyde zunächst mit der doppeltmolaren Menge eines Acetessigsäureniederalkylesters, z.B. nach Knoevenagel unter Verwendung von Piperidin als Kondensationsmittel, im entsprechenden Niederalkanol kondensiert, wobei unter gleichzeitiger Reaktion einer Keto- mit einer Methylgruppe die entsprechenden 2-(4-, 5-, 6- oder 7-benzofuranyl)-4-hydroxy-4-methyl-6-oxo-1,3-cyclohexandicarbonsäure-diniederalkylester entstehen. Aus diesen erhält man durch alkalische Hydrolyse, z.B. Kochen in wässrig-niederalkanolischer Alkalihydroxidlösung, die entsprechenden 3-(4-, 5-, 6- oder 7-Benzofuranyl)-glutarsäuren, aus welchen in üblicher Weise, z.B. durch Kochen in Acetanhydrid, die entsprechenden Anhydride erhalten werden, aus denen man ebenfalls in üblicher Weise durch Umsetzung mit Ammoniak oder Aminen der allgemeinen Formel XII, z.B. durch Erhitzen mit Ammoniumacetat oder Kochen mit einem Amin der allgemeinen Formel XII in Gegenwart von Eisessig, oder mit dem entsprechenden Acetat in einem inerten organischen Lösungsmittel, wie z.B. Toluol, vorzugsweise unter Wasserabscheidung, die von der allgemeinen Formel II umfassten Glutarimide bzw. 2,6-Piperidindione erhält.

Zu Ausgangsstoffen der allgemeinen Formeln III und V sowie zu einem Teil der Ausgangsstoffe der allgemeinen Formeln IV und VII kann man beispielsweise ausgehend von Verbindungen der allgemeinen Formel IX gelangen, in denen $X_g$ Halogen, insbesondere Brom, bedeutet, $R_1$ und $R_2$ die unter der Formel I angegebene Bedeutung haben und der Ring A gegebenenfalls weiter substituiert ist. Von diesen Verbindungen sind einige Vertreter bekannt und weitere analog zu den bekannten, beispielsweise durch Cyclisierung von (Bromphenoxy)-alkanonen oder von Acetalen von α-(Bromphenoxy)-alkanalen, z.B. mit konz. Schwefelsäure, wie das 7-Brom-2,3-dimethylbenzofuran [vgl. Bull. Soc. Chim. France 1966, 586-594], durch Cyclisierung von Estern von 2-(2,3-Dibrompropyl)-bromphenolen, z.B. mit Kaliumhydroxid, wie das 2-Methyl-5-brombenzofuran [vgl. R. Adams et al., J. Am. Chem. Soc. 41, 659, 661 (1919) und L.Claisen, Ber. 53, 322-325 (1920)] oder gegebenenfalls auch durch direkte Bromierung, wie das 4-Brom-5-methoxybenzofuran [vgl. D.S. Noyce, R.W. Nichols, J. Org. Chem. 37, 4313 (1972)] oder durch Cyclisierung von bromsubstituierten 2-Formylphenoxyessigsäuren durch Kochen mit Natriumacetat in Acetanhydrid-Essigsäure, wie z.B. das 5-Brom-6-methoxybenzofuran aus (4-Brom-2-formyl-5-methoxyphenoxy)-essigsäure [vgl. L.R. Worden et al., J. Org. Chem. 34, 2311-2313 (1969)] herstellbar. Die Verbindungen der allgemeinen Formel IX, in denen $X_g$ Halogen, insbesondere Brom, bedeutet, werden zunächst, wie bei Verfahren g) angegeben, in entsprechende Verbindungen mit einen Halogenmagnesium- oder Alkalimetallrest $X_g$, insbe-

sondere einen Brommagnesium- oder Lithiumrest $X_g$ übergeführt und diese metallorganischen Verbindungen analog Verfahren g) <u>in situ</u> mit 4-Piperidinonen, die in 1-Stellung einen Rest entsprechend der Definition für $R_3$ mit Ausnahme von Wasserstoff enthalten, zu Verbindungen der allgemeinen Formel V umgesetzt, die als Rest $X_d$ Hydroxy enthalten, und auch Ausgangsstoffe für das Verfahren e) entsprechend der allgemeinen Formel VII mit Hydroxy als abspaltbaren Rest $X_e$ darstellen. Durch Acylierung solcher Hydroxyverbindungen unter milden Bedingungen, z.B. mit einem Niederalkanoylhalogenid, wie Acetylchlorid in Gegenwart von Pyridin in der Kälte, erhält man entsprechende Verbindungen der allgemeinen Formel V mit einem Acyloxyrest, z.B. dem Acetoxyrest als Rest $X_d$ . Durch Abspaltung von Wasser oder Säure gemäss Verfahren d) erhält man aus den Verbindungen der allgemeinen Formel V Verbindungen der allgemeinen Formel I, in denen $Y_1$ und $Y_2$ eine zusätzliche Bindung bedeuten und die zugleich Ausgangsstoffe der allgemeinen Formel III für das Verfahren b) darstellen.

Ausgangsstoffe der allgemeinen Formel III, in denen $\overline{R_4^b}$ ein 4-Pyridylrest oder ein entsprechender, in 1-Stellung durch einen von Wasserstoff verschiedenen Rest $R_3$ substituierter quaternärer Rest ist, erhält man z.B. durch Umsetzung der vorgenannten Verbindungen der allgemeinen Formel IX, in denen der Rest $X_g$ Halogen, insbesondere Brom, bedeutet, mit einem 4-Metallpyridin, z.B. mit 4-Lithiumpyridin, und gewünschtenfalls anschliessende Quaternierung mit einem Niederalkyl-, Niederalkenyl- oder Niederalkinyl oder Cycloalkylniederalkylhalogenid.

Ausgangsstoffe der allgemeinen Formel IV, in denen Ac der Acylrest eines Kohlensäure- oder Thiokohlensäure-halbesters oder ein Cyanorest oder ein Chlorcarbonylrest ist, lassen sich beispielsweise aus entsprechenden Verbindungen der allgemeinen Formel I, in denen $R_3$ eine leicht abspaltbare Gruppe, wie die Allylgruppe und vor

allem die Methylgruppe ist, durch Umsetzung mit Chlorameisensäure-estern oder -thio-estern, vor allem mit Chlorameisensäure-äthylester, -tert.butylester, -benzylester, -phenylester oder Chlorthioameisensäure-S-methylester, oder mit Bromcyan oder Phosgen in einem inerten organischen Lösungsmittel in der Wärme, z.B. in Toluol bei dessen Siedetemperatur, herstellen.

Anstelle der vorgenannten Kohlensäurederivate kann man z.B. auch Carbonsäurehalogenide, wie z.B. Acetylbromid oder Benzoylchlorid einsetzen, doch erfordert die entsprechende Umsetzung zur Abspaltung der Gruppe $R_3$ meist energischere Bedingungen und ist weniger vollständig als z.B. bei Verwendung von Chlorameisensäureäthylester und insbesondere Bromcyan.

Weitere Ausgangsstoffe der allgemeinen Formel IV kann man beispielsweise durch Umsetzung von vorgenannten Verbindungen der allgemeinen Formel IX, in denen $X_g$ einen Halogenmagnesium- oder Alkalimetallrest, insbesondere Lithiumrest, bedeutet, mit 4-Piperidinonen, die in 1-Stellung einen durch Reduktion oder Solvolyse abspaltbaren Rest, z.B. den Benzylrest, den Benzyloxycarbonylrest oder einen Acylrest, enthalten, herstellen.

Ausgangsstoffe der allgemeinen Formel VII, in denen $X_e$ eine Carboxylgruppe ist, erhält man beispielsweise durch Kondensation eines gegebenenfalls entsprechend der Definition für $R_1$ und $R_2$ sowie im Ring A substituierten 4-, 5-, 6- oder 7-Benzofuranacetonitrils mit einem N,N-Bis-(2-halogenäthyl)-niederalkyl, -niederalkenyl-, -niederalkinyl- -cycloalkyl- oder -cycloalkylniederalkylamin in Gegenwart eines alkalischen Kondensationsmittels, wie z.B. Natrium-oder Lithiumamid oder Natriumhydrid, gefolgt von Hydrolyse oder Solvolyse des erhaltenen, in 1-Stellung durch ein von Wasserstoff verschiedenes $R_3$ substituierten 4-(Benzofuranyl)-isonipecotinonitrils zur entsprechenden Carbonsäure, gegebenenfalls über die üblichen Zwischenstufen

Imidchlorid-hydrochlorid, Imidoniederalkylester und Niederalkylester. Verwendet man für diese Reaktionsfolge anstelle der obengenannten N,N-Bis-(2-halogenäthyl)-verbindungen geeignete N,N-Bis-(2-halogenäthyl)-amide, wie z.B. N,N-Bis-(2-Chloräthyl)-p-toluolsulfonamid, so erhält man durch Hydrolyse bzw. Solvolyse der Nitrilgruppe und anschliessend Decarboxylierung analog Verfahren e) Ausgangsstoffe der allgemeinen Formel IV für das Verfahren c).

Ausgangsstoffe der allgemeinen Formel VIII für die Cyclisierung gemäss Verfahren f) erhält man beispielsweise durch Reduktion der als Zwischenprodukte für die Herstellung von Ausgangsstoffen der allgemeinen Formel II bereits genannten 3-(4-, 5-, 6- oder 7-Benzofuranyl)-glutarsäure-diniederalkylester oder freien Säuren mittels Hydriden, wie z.B. Lithiumaluminiumhydrid oder Diboran, Umsetzung der entstandenen, in 3-Stellung entsprechend substituierten 1,5-Pentandiole mit anorganischen Säurehalogeniden, wie z.B. Thionylchlorid oder Phosphortribromid, zu entsprechend 3-substituierten 1,5-Dihalogenpentanen bzw. mit organischen Sulfonsäurehalogeniden, wie Methansulfonylchlorid oder p-Toluolsulfochlorid, zu entsprechenden 3-substituierten 1,5-Bis-(sulfonyloxy)-pentanen und Umsetzung dieser Verbindungen mit Aminen der allgemeinen Formel XII, vorzugsweise <u>in situ</u>, d.h. unmittelbar vor der Cyclisierung gemäss Verfahren f) in dem für diese Reaktion vorgesehenen Lösungsmittel.

Ausgangsstoffe der allgemeinen Formel XI sind wiederum aus den bereits erwähnten, teilweise bekannten Verbindungen der allgemeinen Formel IX, in denen $X_g$ ein Halogenatom, insbesondere Brom bedeutet und $R_1$ und $R_2$ die unter der Formel I angegebene Bedeutung haben und der Ring A gegebenenfalls weiter substituiert ist, durch Ueberführung in die entsprechenden, ebenfalls unter die allgemeine Formel IX fallenden Halogenmagnesium- bzw. Alkalimetallverbindungen und Umsetzung derselben mit Aceton,

und Dehydratisierung der zunächst entstandenen tertiären Hydroxyverbindungen unter den üblichen Bedingungen der Grignard- bzw. alkalimetallorganischen Reaktionen herstellbar. Ebenfalls in Betracht kommt die Umsetzung von vorzugsweise in 2- und 3-Stellung substituierten 4-, 5-, 6- oder 7-Acetylbenzofuranen, z.B. den im bereits erwähnten Bull. Chem. Soc. Japan 40, 1224-1231 (1967) beschriebenen, mit Methylmagnesiumhalogeniden und wiederum Dehydratisierung der zunächst entstandenen tertiären Hydroxyverbindungen.

Einige Ausgangsstoffe der allgemeinen Formel X, wie z.B. das 4-Chlor-1-methylpiperidin [McElvain, Rorig, J. Am. Chem. Soc. 70, 1826 (1948)] und das 1-Aethyl-4-chlorpiperidin [Paul, Tchelitcheff, Bull. Soc. Chim. France, 1954, 982,983], sind bekannt und weitere analog zu den bekannten Verbindungen herstellbar. Die Ausgangsstoffe der allgemeinen Formeln XII bedürfen keiner weiteren Erläuterung.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Je nach den Verfahrensbedingungen und Ausgangsstoffen erhält man die Verbindungen der allgemeinen Formel I in freier Form oder in der ebenfalls in der Erfindung inbegriffenen Form ihrer Säureadditionssalze. So können beispielsweise basische, neutrale oder gemischte Salze, gegebenenfalls auch Hemi-, Mono-, Sesqui- oder Polyhydrate davon erhalten werden. Die Säureadditionssalze der neuen Verbindungen können in an sich bekannter Weise in die freien Basen übergeführt werden, z.B. mit basischen Mitteln, wie Alkalien oder Ionenaustauschern. Anderseits kann man gewünschtenfalls erhaltene freie Basen mit organischen oder anorganischen Säuren in Säureadditionssalze überführen. Zu deren Herstellung werden insbesondere Säuren verwendet,

die zur Bildung von pharmazeutisch annehmbaren Salzen geeignet sind. Als solche Säuren seien beispielsweise genannt: Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Malein-, Hydroxymalein-, Brenztrauben-, Phenylessig-, Benzoe-, p-Aminobenzoe-, Anthranil-, p-Hydroxybenzoe-, Salicyl-, p-Aminosalicyl-, Embon-, Methansulfon-, Aethansulfon-, Hydroxyäthansulfon- oder Aethylensulfonsäure, Halogenbenzolsulfon-, Toluolsulfon-, oder Naphthalinsulfonsäure, Sulfanilsäure oder andere saure Stoffe, wie Acarbinsäure.

Diese und, sofern sie gut kristallisierbar sind, auch andere Salze können zur Reinigung der neuen Verbindungen verwendet werden, z.B. indem man die freien Verbindungen in ihre Salze überführt, diese isoliert und wieder in die freien Verbindungen überführt. Infolge der engen Beziehung zwischen den neuen Verbindungen in freier Form und in Form ihrer Salze sind im vorausgegangenen und nachfolgend unter den freien Verbindungen sinn- und zweckmässig gegebenenfalls auch die entsprechenden Salze zu verstehen.

Die neuen Verbindungen können, je nach Wahl der Ausgangsstoffe und Arbeitsweisen, als optische Antipoden oder Racemate, oder sofern sie mindestens zwei asymmetrische Kohlenstoffatome enthalten, auch als Racematgemische oder gegebenenfalls auch als Gemische von Stellungsisomeren vorliegen.

Erhaltene Racematgemische können auf Grund der physikalisch-chemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden. Ebenfalls vorzugsweise durch fraktionierte Kristallisation können Gemische von Stellungsisomeren

getrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säure, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, 0,0-Di-p-toluylweinsäure, Aepfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, nach denen man von einer, auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen man eine Reaktionskomponente gegebenenfalls in Form eines Derivates, z.B. eines Salzes, verwendet.

Die Verbindungen der allgemeinen Formel I sowie ihre pharmazeutisch annehmbaren Säureadditionssalze werden in Form von pharmazeutischen Zusammensetzungen vorzugsweise peroral oder rektal verabreicht, können jedoch in Form wässriger Lösungen ihrer Säureadditionssalze auch parenteral verabreicht werden.

Die täglichen Dosen für Warmblüter bewegen sich zwischen 0,03 und 3 mg/kg und liegen für Warmblüter von

ca. 70 kg Körpergewicht vorzugsweise zwischen 2,5 und 50 mg. Geeignete Doseneinheitsformen, wie Dragées, Tabletten, Suppositorien, enthalten vorzugsweise 1 bis 25 mg eines erfindungsgemässen Wirkstoffes, d.h. einer Verbindung der allgemeinen Formel I oder eines pharmazeutisch annehmbaren Säureadditionssalzes einer solchen. Zur Herstellung von erfindungsgemässen pharmazeutischen Zusammensetzungen kombiniert man den Wirkstoff mit mindestens einem pharmazeutischen Trägerstoff. Zur Herstellung der vorgenannten, oral verabreichbaren Doseneinheitsformen verarbeitet man z.B. den Wirkstoff mit festen pulverförmigen Trägerstoffen, wie Lactose, Saccharose, Sorbit, Amnnit, Stärken, wie Kartoffelstärke, Maisstärke oder Amylopektin, ferner Laminariapulver oder Citruspulpenpulver, Cellulosederivaten oder Gelatine, gegebenenfalls unter Zusatz von Gleitmitteln, wie Magnesium- oder Calciumstearat oder Polyäthylenglykolen, zu Tabletten oder zu Dragées-Kernen. Letztere überzieht man beispielsweise mit konzentrierten Zuckerlösungen, welche z.B. noch arabischen Gummi, Talk und/oder Titandioxid enthalten können, oder mit einem in leichtflüchtigen organischen Lösungsmitteln oder Lösungsmittelgemischen gelösten Lack. Diesen Ueberzügen können Farbstoffe zugefügt werden, z.B. zur Kennzeichnung verschiedener Wirkstoffdosen. Als weitere orale Doseneinheitsformen eignen sich Steckkapseln aus Gelatine sowie weiche, geschlossene Kapseln aus Gelatine und einem Weichmacher, wie Glycerin. Die ersteren enthalten den Wirkstoff vorzugsweise als Granulat in Mischung mit Gleitmitteln, wie Talk oder Magnesiumstearat, und gegebenenfalls Stabilisatoren, wie Natriummetabisulfit oder Ascorbinsäure.

Als Doseneinheitsformen für die rektale Anwendung kommen z.B. Suppositorien in Betracht, welche aus einer Kombination eines Wirkstoffes mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner eignen sich auch Gelatine-Rektalkapseln, welche aus einer Kombination des Wirkstoffes mit einer Grundmasse bestehen. Als Grundmasse eignen sich z.B. flüssige Triglyceride, Polyäthylenglykole oder Paraffinkohlenwasserstoffe.

Ampullen zur parenteralen, insbesondere intramuskulären Verabreichung enthalten vorzugsweise ein wasserlösliches Salz eines Wirkstoffes in einer Konzentration von vorzugsweise 0,5-5%, gegebenenfalls zusammen mit geeigneten Stabilisierungsmitteln und Puffersubstanzen, in wässeriger Lösung.

Die folgenden Vorschriften sollen die Herstellung von Tabletten, Dragées, Suppositorien und Ampullen näher erläutern:

a) 100,0 g 4-(2,3-Dimethyl-5-benzofuranyl)-piperidin-hydrochlorid werden mit 450 g Lactose und 292 g Kartoffelstärke vermischt, die Mischung mit einer alkoholischen Lösung von 8 g Gelatine befeuchtet und durch ein Sieb granuliert. Nach dem Trocknen mischt man 60 g Kartoffelstärke, 60 g Talk, 10 g Magnesiumstearat und 20 g hochdisperses Siliciumdioxid zu und presst die Mischung zu 10'000 Tabletten von je 100 mg Gewicht und 10 mg Wirkstoffgehalt, die gewünschtenfalls mit Teilkerben zur feineren Anpassung der Dosierung versehen sein können.

b) 12,5 g 4-(2,3-Dimethyl-6-benzofuranyl)-piperidin-hydrochlorid werden mit 16 g Maisstärke und 6 g hochdispersem Siliciumdioxid gut vermischt. Die Mischung wird mit einer Lösung in ca. 70 ml Isopropylalkohol befeuchtet und durch ein Sieb mit 1,2 mm Maschenweite granuliert. Das Granulat wird ca. 14 Stunden getrocknet und dann durch ein Sieb mit 1,2 bis 1,5 mm Maschenweite geschlagen. Hierauf wird es mit 16 g Maisstärke, 16 g Talk und 2 g Magnesiumstearat vermischt und zu 1000 Dragée-Kernen gepresst. Diese werden mit einem konzentrierten Sirup von 2 g Lacca, 7,5 g arabischen Gummi, 0,15 g Farbstoff, 2 g hochdispersem Siliciumdioxid, 25 g Talk und 53,35 g Zucker überzogen und getrocknet. Die erhaltenen Dragées wiegen je 158,5 mg und enthalten je 12,5 mg Wirkstoff.

c) 25,0 g 4-(6,7,8,9-Tetrahydro-2-dibenzofuranyl)-piperidin-hydrochlorid und 1975 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2 g gegossen. Sie enthalten je 25 mg Wirkstoff.

d) 12,5 g 4-(2,3-Dimethyl-5-benzofuranyl)-piperidin-hydrochlorid werden in 1 Liter bidestilliertem pyrogenfreiem Wasser gelöst und die Lösung in 1000 Ampullen abgefüllt und sterilisiert. Eine Ampulle enthält eine 2,5%-ige Lösung von 12,5 mg Wirkstoff.

Die nachfolgenden Beispiele erläutern die Herstellung der neuen Verbindungen der allgemeinen Formel I und von bisher nicht bekannten Ausgangsstoffen näher, sollen jedoch den Umfang der Erfindung in keiner Weise beschränken. Die Temperaturen sind in Celsiusgraden angegeben.

Beispiel 1

Zu einer im Eis-Wasser-Bad gekühlten und unter Stickstoff befindlichen Suspension von 2,8 g (0,075 Mol) Lithiumaluminiumhydrid in 50 ml absolutem Tetrahydrofuran tropft man unter Rühren langsam 6,1 g (0,025 Mol) 4-(2,3-Dimethyl-5-benzofuranyl)-2-piperidinon, teilweise gelöst bzw. suspendiert in 50 ml absolutem Tetrahydrofuran, innerhalb ca. 15 Minuten zu. Dabei wird heftiges Schäumen und ein Temperaturanstieg auf ca. 20° beobachtet. Nach erfolgter Zugabe entfernt man das Kühlbad und kocht das graue Reaktionsgemisch 6 Stunden unter Rückfluss. Danach kühlt man das Reaktionsgemisch im Eis-Wasser-Bad und zersetzt überschüssiges Reduktionsmittel vorsichtig mit 8.4 ml Wasser und 5,6 ml 2-n. Natronlauge. Der dabei entstehende Komplex wird noch 15 Minuten gerührt, danach abgenutscht und zweimal mit ca. 20 ml absolutem Tetrahydrofuran gewaschen. Das Filtrat wird im Rotationsverdampfer auf dem Wasserbad vollständig eingedampft.

Das erhaltene Rohprodukt wird in ca. 15 ml Methanol aufgenommen und mit ca. 6-n. methanolischer Salzsäure auf den pH-Wert 3 eingestellt. Sodann fällt man durch Zugabe von Aether das rohe Hydrochlorid aus. Dieses wird abgenutscht, mit Aethanol/Aether (1:9) gewaschen und getrocknet. Nach Umkristallisation aus Methanol/Aether schmilzt das so erhaltene 4-(2,3-Dimethyl-5-benzofuranyl)-piperidin-hydrochlorid bei 269-270°.

Der Ausgangsstoff wird wie folgt hergestellt:

a) 21,8 g (0,1 Mol) 2,3-Dimethyl-5-benzofurancarbonsäure-äthylester [hergestellt nach Y. Kawase und M Takashima, Bull. Chem. Soc. Japan 40, 1224 (1967)], werden in 50 ml wasserfreiem Tetrahydrofuran gelöst. Diese Lösung wird unter Rühren tropfenweise zu einer mit Eiswasser gekühlten Suspension von 3,8 g (0,1 Mol) Lithiumaluminiumhydrid in

150 ml wasserfreiem Tetrahydrofuran gegeben. Das Reaktionsgemisch wird 2 Stunden bei 0° gerührt; anschliessend lässt man es auf Raumtemperatur erwärmen. Die erhaltene Suspension wird mit Eis-Wasser-Gemisch gekühlt und überschüssiges Reduktionsmittel durch tropfenweise Zugabe von 11,4 ml Wasser und 7,6 ml 2-n. Natronlauge zersetzt. Die dabei entstehende Suspension eines weissen Komplexes wird 15 Minuten weitergerührt, danach wird der Komplex abgenutscht und zweimal mit je ca. 50 ml Tetrahydrofuran nachgewaschen. Die vereinigten Filtrate werden im Rotationsverdampfer auf dem Wasserbade zur Trockene eingedampft. Das dabei anfallende farblose Oel kristallisiert beim Stehenlassen durch, Schmelzpunkt des Rohproduktes 70-71°. Dessen Reinheit genügt für seine direkte Weiterverwendung.

b) Eine Lösung von 158,2 g (0,9 Mol) 2,3-Dimethyl-5-benzofuranmethanol in 1500 ml Toluol wird mit 400 g Mangandioxid während 3 Tagen bei Raumtemperatur gerührt. Danach wird das Oxidationsmittel über Diatomeenerde abfiltriert, das Filtergut mit Toluol nachgewaschen und das Filtrat im Rotationsverdampfer vollständig eingedampft. Der schwach gelbliche Rückstand von 2,3-Dimethyl-5-benzofurancarboxaldehyd kristallisiert spontan, Smp. 80-81°. Eine aus Hexan umkristallisierte Probe schmilzt bei 81-82°. Das Produkt kann ohne Reinigung weiterverwendet werden.

c) Zu einer Emulsion, bestehend aus 300 ml Dichlormethan, 170 ml 50%-iger Natronlauge und 11 g einer 40%-igen wässrigen Lösung von Tetrabutylammoniumhydroxid, wird unter starkem Rühren bei Raumtemperatur innerhalb 30 Minuten eine Lösung von 52,2 g (0,3 Mol) 2,3-Dimethyl-5-benzofurancarboxaldehyd und 55,8 g (0,315 Mol) Diäthoxyphosponoacetonitril in 50 ml Dichlormethan zugetropft. Das Reaktionsgemisch erwärmt sich von selbst auf ca. 35°. Man rührt das Reaktionsgemisch nach beendeter Zugabe 60 Minuten bei Raumtemperatur, giesst es danach auf 200 ml Wasser, trennt die

organische Schicht ab und extrahiert die wässrige Phase noch zweimal mit Dichlormethan. Die vereinigten Dichlormethanphasen werden dreimal mit gesättigter Natriumchloridlösung gewaschen, über Magnesiumsulfat getrocknet und im Rotationsverdampfer eingeengt. Dabei tritt bei einem Totalvolumen von ca. 100 ml spontane Kristallisation ein. Man gibt deshalb noch ca. 100 ml Hexan zu, lässt weiter kristallisieren und saugt danach die entstandenen Kristalle des 2,3-Dimethyl-5-benzofuranacrylonitril ab. Durch mehrmaliges Kristallisieren der Mutterlauge lässt sich noch weitere Reinsubstanz gewinnen. Die Hauptmenge des anfallenden Produktes besteht aus dem trans-Isomeren. Die vierte Kristallfraktion enthält ca. 50 % cis-Isomeres, wie aus dem Kernresonanzspektrum geschlossen werden kann. Die Mutterlauge der vierten Kristallisation besteht ebenfalls aus einem Gemisch der cis- und trans-Isomeren im Verhältnis von ungefährt 1:1. Für den weiteren Reaktionsverlauf sört die Anwesenheit des cis-Isomeren nicht.

d) Eine Lösung von 30,0 g (0,15 Mol) 2,3-Dimethyl-5-benzofuranacrylonitril und 24.8 g (0,155 Mol) trockenem Malonsäurediäthylester in 70 ml absolutem Aethanol wird portionenweise mit 3,5 g (0,15 Mol) Natrium in kleinen Stücken versetzt. Durch die exotherme Reaktion kommt das Reaktionsgemisch zum Sieden. Nach beendeter Zugabe (Dauer ca. 15 Minuten) wird das gelbe Reaktionsgemisch noch 60 Minuten unter Rückfluss gekocht. Dann wird das Reaktionsgemisch gekühlt und mit 9 ml (1 Aequivalent) Eisessig und 100 ml Wasser versetzt. Das erhaltene Gemisch giesst man auf 1 Liter Aether, gibt 400 ml gesättigte Natriumchloridlösung zu und trennt die organische Phase ab. Die wässrige Phase wird anschliessend noch zweimal mit je 400 ml Aether extrahiert. Die organischen Phasen werden dreimal mit gesättigter Natriumchloridlösung, einmal mit

wässriger 1-n. Kaliumbicarbonatlösung und nochmals mit gesättigter Natriumchloridlösung gewaschen. Hierauf werden die organischen Phasen vereinigt, über Magnesiumsulfat getrocknet und im Rotationsverdampfer eingedampft. Das zurückbleibende Oel kristallisiert allmählich. Es wird in 80 ml heissem Isopropanol aufgenommen und in der Wärme mit ca. 150 ml Ligroin versetzt. Aus der Lösung kristallisiert beim langsamen Kühlen der [2-Cyano-1-(2,3-dimethyl-5-benzofuranyl)-äthyl]-malonsäure-diäthylester vom Smp. 84-86°. Aus der Mutterlauge kann analog noch weitere kristallisierte Substanz erhalten werden. Eine aus Isopropanol-Ligroin umkristallisierte Probe des ersten Kristallisates schmilzt bei 86.5-87°

e) Eine Lösung von 35,7 g [2-Cyano-1-[2,3-dimethyl-5-benzofuranyl)-äthyl]-malonsäure-diäthylester in 180 ml absolutem Aethanol wird in Gegenwart von 8 g Raney-Nickel unter 120 bar Anfangsdruck während 13 Stunden bei 70-80° hydriert. Danach wird der Katalysator über Diatomeenerde filtriert und das Filtrat im Rotationsverdampfer auf dem Wasserbad vollständig eingedampft. Auf eine Reinigung des langsam kristallierenden, rohen 4-(2,3-Dimethyl-5-benzofuranyl)-2-oxo-nipecotinsäure-äthylesters wird wegen den dabei auftretenden Schwierigkeiten infolge des vorliegenden Diastereomerengemisches verzichtet. Das Rohprodukt wird deshalb ohne weitere Reinigung weiterverwendet. Im Dünnschichtchromatogramm auf Silikagel mit dem Laufmittel Chloroform + 2 % Methanol ist der Hauptfleck bei Rf. ca. 0,2 sichtbar (Jod), 3 schwache Nebenflecken sind bei Rf. 0,15; 0,3 und 0,7 erkennbar.

f) Eine Lösung von 31,5 g (0,1 Mol) 4-(2,3-Dimethyl-5-benzofuranyl)-2-oxo-nipecotinsäure-äthylester in 100 ml Aethanol wird mit 50 ml 2-n. Natriumcarbonatlösung und 50 ml 2-n. Natronlauge versetzt. Hierauf erwärmt man das Reaktionsgemisch und kocht es 30 Minuten unter Rückfluss. Das

Reaktionsgemisch wird sodann gekühlt und mit 2-n. Salzsäure bis zum pH-Wert von ca. 4 versetzt. Dabei fällt ein kristalliner Niederschlag aus, der abgenutscht, mit kaltem Wasser gewaschen und anschliessend getrocknet wird. Die erhaltene 4-(2,3-Dimethyl-2-benzofuranyl)-2-oxo-nipecotinsäure kann ohne Reinigung weiterverwendet werden.

g) Eine Suspension von 28,7 g (0,1 Mol) 4-(2,3-Dimethyl-5-benzofuranyl)-2-oxo-nipecotinsäure in 120 ml Toluol wird allmählich zum Sieden unter Rückfluss erhitzt. Bei ca. 100° Innentemperatur erfolgt Kohlendioxid-Entwicklung und sukzessives Auflösen der suspendierten Substanz. Nach ca. 10 Minuten wird kein Gasabgang mehr beobachtet. Man kocht die entstandene gelbe Lösung noch 20 Minuten unter Rückfluss und kühlt sie dann ab, wobei sich allmählich ein kristalliner Niederschlag abscheidet. Es werden 200 ml Hexan zugegeben, um die Kristallisation zu vervollständigen. Hierauf werden die Kristalle abgesaugt und mit Toluol gewaschen. Das erhaltene 4-(2,3-Dimethyl-5-benzofuranyl)-2-piperidinon schmilzt bei 168-170°. Aus der Mutterlauge erhält man durch Kristallisation aus Aethylacetat/Hexan noch weiteres Rohprodukt. Eine aus Aethylacetat/Hexan umkristallisierte Probe schmilzt bei 172,5-173,5°. Das Rohprodukt ist für eine Weiterverwendung genügend rein.

Beispiel 2

0,95 g (0,025 Mol) Lithiumaluminiumhydrid werden in 10 ml absolutem Tetrahydrofuran suspendiert. Zu dieser Suspension tropft man unter Kühlung mit Eis-Wasser-Gemisch innerhalb 20 Minuten 5,14 g (0,02 Mol) 1-Methyl-4-(2,3-dimethyl-5-benzofuranyl)-2-piperidinon in 40 ml absolutem Tetrahydrofuran gelöst zu. Man rührt das Gemisch 30 Minuten bei 0-5° und lässt es anschliessend auf Raumtemperatur erwärmen.

Nach 30 Minuten Reaktionszeit bei Raumtemperatur kühlt man das Reaktionsgemisch und zerstört überschüssiges Reduktionsmittel unter Eis-Wasser-Kühlung mit 3 ml Wasser und 2 ml 2-n. Natronlauge. Die entstandene Suspension wird 15 Minuten gerührt, dann genutscht und das Filtergut mit Tetrahydrofuran nachgewaschen. Das Filtrat dampft man im Rotationsverdampfer vollständig ein. Den gelben, klaren, öligen Rückstand nimmt man in absolutem Aethanol auf, säuert mit ca. 6-n. ätherischer Salzsäure auf den pH-Wert 4 an und fällt durch Zugabe von Aether das rohe Hydrochlorid aus. Das so erhaltene 1-Methyl-4-(2,3-dimethyl-5-benzofuranyl)-piperidin-hydrochlorid schmilzt bei 236-238°. Eine aus Aethanol/Aether umkristallisierte Probe schmilzt bei 240-242°.

Der Ausgangsstoff wird wie folgt hergestellt:
a) 1,15 g (0,024 Mol) einer 50%-igen Suspension von Natriumhydrid in Mineralöl werden dreimal mit je ca. 20 ml Pentan ölfrei gewaschen. Das verbleibende Natriumhydrid wird unter Stickstoff bei Raumtemperatur mit 15 ml absolutem Dimethylsulfoxid versetzt. Zur Lösung von Dimethylsulfoxid-Natrium tropft man eine Lösung von 4,6 g (0,02 Mol) 4-(2,3-Dimethyl-5-benzofuranyl)-2-piperidinon [vgl. Beispiel 1a) bis g)] in 20 ml Dimethylsulfoxid innerhalb ca. 15 Minuten bei Raumtemperatur zu, wobei unter Aufschäumen eine bräunliche Suspension entsteht. Diese wird bei Raumtemperatur tropfenweise mit 3,4 g (0,024 Mol) Methyljodid versetzt und alsdann noch 90 Minuten bei Raumtemperatur gerührt. Dann wird das Reaktionsgemisch auf ca. 150 ml Wasser gegossen und dreimal ausgeäthert. Die organischen Phasen werden mehrmals mit Wasser und dann mit gesättigter Natriumchloridlösung gewaschen, vereinigt, über Magnesiumsulfat getrocknet und im Rotationsverdampfer eingedampft. Das in Form von gelblichen Eiskristallen zu-

rückbleibende 1-Methyl-4-(2,3-dimethyl-5-benzofuranyl)-2-piperidinon schmilzt bei 142-144°. Dieses Produkt ist von genügender Reinheit und kann direkt für die nachfolgende Reduktion verwendet werden.

Beispiel 3

Analog Beispiel 1 wird durch Reduktion von 6,1 g (0,025 Mol) 4-(2,3-Dimethyl-6-benzofuranyl-2-piperidinon das 4-(2,3-Dimethyl-6-benzofuranyl)-piperidin-hydrochlorid vom Smp. 275-277° (aus Methanol/Aether) erhalten.

Der Ausgangsstoff wird wie folgt hergestellt:

a) Analog Beispiel 1a) erhält man durch Reduktion von 21,8 g (0,1 Mol) 2,3-Dimethyl-6-benzofurancarbonsäure-äthylester [hergestellt nach Y. Kawase und M. Takashima, Bull. Chem.Soc., Japan 40, 1224 (1967)] das 2,3-Dimethyl-6-benzofuranmethanol vom Smp. 85-85,5° (aus Aether-Hexan).

b) Analog Beispiel 1b) erhält man den 2,3-Dimethyl-6-benzofurancarboxaldehyd vom Smp. 73-74° (aus Hexan) durch Oxidation von 158,2 g (0,9 Mol) 2,3-Dimethyl-6-benzofuranmethanol.

c) 2,3-Dimethyl-6-benzofuranacrylonitril wird aus 52,2 g (0,3 Mol) 2,3-Dimethyl-6-benzofurancarboxaldehyd auf analoge Weise wie im Beispiel 1c) beschrieben hergestellt. Das Reaktionsprodukt besteht aus einem cis/trans Gemisch im Verhältnis 3:7, wobei der Schmelzpunkt (aus Isopropanol) der reinen Isomeren 125-128° für das cis-Isomere und 134-136° für das trans-Isomere beträgt.

d) In analoger Weise wie im Beispiel 1d) beschrieben wird ausgehend von 30,0 g (0,15 Mol) 2,3-Dimethyl-6-benzofuranacrylonitril der 2-Cyano-1-(2,3-dimethyl-6-benzofuranyl)-äthyl]-malonsäure-diäthylester hergestellt. Der Schmelzpunkt einer dünnschichtchromatographisch einheitlichen Probe beträgt 118-120° (aus Isopropanol/Hexan). Weniger reine aber für die Weiterverwendung geeignete Proben zeigen

Schmelzpunkte zwischen 110° bis 120°.
e) Aus 35,7 g (0,1 Mol) [2-Cyano-1-(2,3-dimethyl-6-benzofuranyl)-äthyl]-malonsäure-diäthylester wird in analoger Weise wie im Beispiel 1e) beschrieben, aber bei 90-100° Reaktionstemperatur, der 3-(2,3-Dimethyl-6-benzofuranyl)-2-oxonipecotinsäure-äthylester hergestellt. Die Reinsubstanz schmilzt bei 157-159° (aus Dichlormethan/Hexan). Abweichungen im Schmelzpunkt nach unten sind auf unterschiedliche Diastereomerenanteile im Kristallisat zurückzuführen.
f) Eine Lösung von 3,15 g (0,01 Mol) 4-(2,3-Dimethyl-6-benzofuranyl)-2-oxonipecotinsäure-äthylester, 0,64 g (0,011 Mol) Natriumchlorid und 0,36 ml (0,02 Mol) Wasser in 10 ml Dimethylsulfoxid wird 3 1/4 Stunden auf 150° erhitzt. Die Lösung verfärbt sich schwach bräunlich und es entwickelt sich Kohlendioxid. Danach kühlt man das Reaktionsgemisch ab, giesst es auf ca. 50 ml Wasser und extrahiert mit Dichlormethan. Die organische Phase wird mehrmals mit Wasser gewaschen, dann über Natriumsulfat getrocknet und im Rotationsverdampfer vollständig eingedampft. Man erhält das 4-(2,3-Dimethyl-6-benzofuranyl)-2-piperidinon als schwach gelbliche Kristalle vom Smp. 195-197°, deren Reinheit für eine direkte Weiterverwendung ausreicht. Eine aus Aethylacetat umkristallisierte Probe schmilzt bei 200-201°.

Beispiel 4

Zu einer Suspension von 0,76 g (0,020 Mol) Lithiumaluminiumhydrid in 100 ml Aether gibt man unter Kühlung im Eis-Wasser-Bad portionenweise 2,57 g (0,010 Mol) 3-(2,3-Dimethyl-5-benzofuranyl)-glutarimid. Nach erfolgter Zugabe lässt man das Reaktionsgemisch sich auf Raumtemperatur erwärmen und kocht es hierauf 2 Stunden unter Rückfluss. Die erhaltene sandfarbene Suspension kühlt man im Eis-Wasserbad, gibt

nacheinander langsam 1,52 ml 2-n. Natronlauge und 2,2 ml Wasser zu und rührt die weisse Suspension 30 Minuten bei derselben Temperatur. Danach filtriert man das Reaktionsgemisch und wäscht den Filterrückstand mehrmals mit Methylenchlorid nach. Die vereinigten Filtrate werden im Rotationsverdampfer eingedampft und der Rückstand in Aethanol gelöst. Durch Zufügen von ätherischer Chlorwasserstofflösung fällt man das 4-(2,3-Dimethyl-5-benzofuranyl)-piperidin-hydrochlorid aus und filtriert es ab, Smp. 267-269°.

In analoger Weise erhält man unter Verwendung von 2,57 g (0,010 Mol) 3-(2,3-Dimethyl-6-benzofuranyl)-glutarimid das 4-(2,3-Dimethyl-6-benzofuranyl)-piperidin-hydrochlorid vom Smp. 277° (aus Methanol-Aether).

Die Ausgangsstoffe können wie folgt hergestellt werden:

a) Eine Lösung von 34,8 g (0,2 Mol) 2,3-Dimethyl-5-benzofurancarboxaldehyd [vgl. Beispiel 1a) und b)] in 100 ml abs. Aethanol wird mit 52,6 g (0,404 Mol) Acetessigsäureäthylester und 3,6 ml Piperidin versetzt. Die gelbliche Lösung lässt man bei Raumtemperatur 48 Stunden stehen. Dabei bildet sich allmählich ein kristalliner Niederschlag Dieser wird zerstossen, abgenutscht, mit Aethanol gewaschen und im Hochvakuum getrocknet. Der so als weisse Kristalle erhaltene 2-(2,3-Dimethyl-5-benzofuranyl-4-hydroxy-4-methyl-6-oxo-1,3-cyclohexandicarbonsäure-diäthylester schmilzt bei 156-158,5°.

In analoger Weise erhält man unter Verwendung von 34,8 g (0,2 Mol) 2,3-Dimethyl-6-benzofurancarboxaldehyd [vgl. Beispiel 3a) und b) den 2-(2,3-Dimethyl-6-benzofuranyl)-4-hydroxy-4-methyl-6-oxo-1,3-cyclohexandicarbonsäurediäthylester vom Smp. 183-185°.

b) Zu einer Lösung von 160 g Natriumhydroxid (4 Mol) im

475 ml Wasser und 200 ml Aethanol gibt man bei 90° portionenweise 83,2 g (0,2 Mol) des ersten gemäss a) erhaltenen Diäthylesters. Die entstehende gelbe Suspension wird 30 Minuten auf 90° erwärmt. Dann kühlt man das Reaktionsgemisch auf Raumtemperatur ab, verdünnt es mit Wasser auf ein Totalvolumen von ca. 1,5 Liter und stellt es unter Eis-Wasser-Kühlung mit konz. Salzsäure kongosauer. Der dabei ausfallende weisse Niederschlag wird abgenutscht, mit Wasser gewaschen und getrocknet, wobei die 3-(2,3-Dimethyl-5-benzofuranyl)-glutarsäure als schwach sandfarbene Kristalle vom Smp. 203-205° (nach Umkristallisation aus Aethanol-Wasser 205-206°) erhalten wird.

In analoger Weise erhält man unter Verwendung von 83,2 g (0,2 Mol) des zweiten gemäss a) hergestellten Diäthylesters die 3-(2,3-Dimethyl-6-benzofuranyl)-glutarsäure vom Smp. 190-192°.

c) 27,6 g (0,1 Mol) 3-(2,3-Dimethyl-5-benzofuranyl)-glutarsäure werden eine Stunde in 120 ml Acetanhydrid unter Rückfluss gekocht. Dann werden das überschüssige Reagens und die entstandene Essigsäure im Rotationsverdampfer abgedampft. Der bräunliche Rückstand wird in Aether gelöst und mit Hexan versetzt,bis Kristallisation eintritt. Die Kristalle werden abgenutscht, mit Aether /Hexan gewaschen und getrocknet. Das so als schwach sandfarbene Kristalle erhaltene 3-(2,3-Dimethyl-5-benzofuranyl)-glutarsäureanhydrid schmilzt bei 107-110° und nach Umkristallisation aus Methylenchlorid-Hexan bei 111-112°.

In analoger Weise erhält man unter Verwendung von 27,6 g (0,1 Mol) 3-(2,3-Dimethyl-6-benzofuranyl)-glutarsäure das 3-(2,3-Dimethyl-6-benzofuranyl)-glutarsäureanhydrid vom Smp. 145-147°.

d) 25,8 g (0,1 Mol) 3-(2,3-Dimethyl-5-benzofuranyl)-glutarsäureanhydrid versetzt man mit 77 g (4 Mol) Ammoniumacetat, mischt das Gemenge gut durch und erhitzt es 5 Stunden auf 160°. Dann kühlt man es, giesst es auf Eis-Wasser-Gemisch und extrahiert das Ganze mit Aethylenchlorid. Die organische Phase wird neutral gewaschen, getrocknet und eingedampft. Das erhaltene sandfarbene Pulver kristallisiert man aus Dioxan-Hexan, wobei man das 3-(2,3-Dimethyl-5-benzofuranyl)-glutarimid vom Smp. 183-185° erhält.

In analoger Weise erhält man unter Verwendung von 25,8 g (0,1 Mol) 3-(2,3-Dimethyl-6-benzofuranyl)-glutarsäureanhydrid das 3-(2,3-Dimethyl-6-benzofuranyl)-glutarimid vom Smp. 252-254°.

Beispiel 5

Analog Beispiel 4 erhält man unter Verwendung von 2,57 g (0,010 Mol) 3-(2,3-Dimethyl-4-benzofuranyl)-glutarimid das 4-(2,3-Dimethyl-4-benzofuranyl)-piperidin-hydrochlorid vom Smp. 340-342° (aus Methanol-Aether).

Der Ausgangsstoff kann wie folgt hergestellt werden:

a) Analog Beispiel 1a) erhält man durch Reduktion von 19,0 g (0,1 Mol) 2,3-Dimethyl-4-benzofurancarbonsäure [hergestellt gemäss Y. Kawasawe und M. Takashima, Bull. Chem. Soc. Japan 40, 1224-1231 (1967)] das 2,3-Dimethyl-4-benzofuranmethanol als direkt weiterverwendbares Rohprodukt.

b) Analog Beispiel 1b) wird unter Verwendung von 158,2 g (0,9 Mol) 2,3-Dimethyl-4-benzofuranmethanol der 2,3-Dimethyl-4-benzofurancarboxaldehyd hergestellt.

c) bis f) Analog Beispiel 4a) bis 4d) erhält man unter Verwendung der jeweils gleichen Mengen der entsprechenden isomeren Ausgangsstoffe die nachstehend angegebenen Zwischenprodukte, nämlich

e) 2-(2,3-Dimethyl-4-benzofuranyl)-4-hydroxy-4-methyl-6-oxo-1,3-cyclohexandicarbonsäure-diäthylester vom Smp. 179-180°;

d) 3-(2,3-Dimethyl-4-benzofuranyl)-glutarsäure vom Smp. 210-211,5°;

e) 3-(2,3-Dimethyl-4-benzofuranyl)-glutarsäureanhydrid vom Smp. 138-139°; und

f) 3-(2,3-Dimethyl-4-benzofuranyl)-glutarimid vom Smp. 214-215° (aus Dioxan-Aether).

Beispiel 6

Analog Beispiel 4 erhält man unter Verwendung von 2,57 g (0,010 Mol) 3-(2,3-Dimethyl-7-benzofuranyl)-glutarimid das 4-(2,3-Dimethyl-7-benzofuranyl)-piperidin-hydrochlorid vom Smp. 287-288° (aus Methanol-Aether).

Der Ausgangsstoff kann wie folgt hergestellt werden:

a) Eine Lösung von 100 g (0,6 Mol) Salicylsäure-äthylester in 300 ml Aceton wird mit 135 g (0,6 Mol) p-Toluolsulfonsäure-(1-methyl-2-propinyl)-ester [H. Schmid et al. Helv., Chim. Acta 55, 1133(1972)] und 207g(1,5 Mol) Kaliumcarbonat versetzt. Dieses Reaktionsgemisch kocht man 72 Stunden unter Rückfluss. Dann wird das bräunliche Reaktionsgemisch abgekühlt und filtriert, das Filtergut wird mit Aceton nachgewaschen und die vereinigten Filtrate werden im Rotationsverdampfer eingedampft. Das erhaltene braune Oel wird in Aether gelöst und die Lösung dreimal mit 2-n. Natronlauge und dann mit gesättigter Natriumchloridlösung neutral gewaschen. Die ätherische Lösung wird getrocknet und im Rotationsverdampfer eingedampft. Der Rückstand wird im Hochvakuum fraktioniert, wobei der o-(1-Methyl-2-propinyloxy)-benzoesäure-äthylester vom Kp. 73-75°/0,005 Torr erhalten wird.

b) 17 g Quecksilber(II)-sulfat löst man in 215 ml Wasser und 55 ml Aethanol erwärmt die Lösung auf 40° und tropft bei dieser Temperatur innerhalb etwa 20 Minuten 98,0 g (0,45 Mol) o-(1-Methyl-2-propinyloxy)-benzoesäure-äthylester zu. Nach beendeter Zugabe rührt man das Reaktionsgemisch eine Stunde bei 45-50°. Danach wird es gekühlt und zur Abtrennung des ausgeschiedenen Quecksilber durch Diatomeenerde filtriert und das Filtergut mit Aether nachgewaschen. Das Filtrat wird getrocknet und eingedampft. Das zurückbleibende rote Oel löst man in 250 ml Methylenchlorid und gibt 5 ml 30 %-ige wässrige Wasserstoffperoxidlösung zu, wobei sofort ein roter Niederschlag von Quecksilber(II)-oxid ausfällt. Nach 30 Minuten filtriert man das Ganze durch Diatomeenerde, schüttelt das Filtrat mehrmals mit Wasser, bis der Kaliumjodid-Test negativ ist, trocknet es und dampft es im Rotationsverdampfer ein. Der Rückstand wird im Hochvakuum fraktioniert, wobei man den o-(1-Methyl-2-oxopropoxy-benzoesaure-äthylester von Kp. 86-87°/0,01 Torr erhält.

c) 35,4 g (0,15 Mol) des obigen Aethylesters und 15 ml abs. Aethanol werden im Eis-Wasser-Bad gekühlt. Dann tropft man innerhalb 20 Minuten 34 g (18,5 Mol) konzentrierte Schwefelsäure zu. Die Temperatur steigt dabei an und wird auf 10-15° gehalten. Hierauf lässt man das Reaktionsgemisch sich auf Raumtemperatur erwärmen (Dauer ca. 15 Minuten) und erhitzt es dann 60 Minuten auf 55° Innentemperatur. Das Reaktionsgemisch von rot-brauner Farbe wird hierauf gekühlt und dann auf ein Gemisch von etwa 50 g Eis und 100 ml Aethylacetat gegossen. Die abgetrennte organische Phase wird zunächst dreimal mit Wasser, dann dreimal mit wässriger 1-n. Natriumbicarbonatlösung und schliesslich mit gesättigter Natriumchloridlösung gewaschen, dann mit Natriumsulfat getrocknet und im Rotationsverdampfer eingedampft. Das zurückbleibende gelbe Oel wird im Hochvakuum destilliert, wobei man den 2,3-Dimethyl-7-benzofurancarbonsäure-äthylester als farbloses, beim Stehenlassen schwach gelblich werdendes Oel vom Kp.93,5-95°/0,005 Torr erhält.

d) 21,8 g (0,1 Mol) des gemäss c) erhaltenen Esters werden in 50 ml Tetrahydrofuran gelöst und die Lösung portionenweise unter Eiskühlung und Stickstoffatmosphäre zu einer Suspension von 3,7 g Lithiumaluminiumhydrid in 150 ml Tetrahydrofuran gegeben. Nach beendeter Zugabe entfernt man das Kühlbad und rührt das Gemisch noch 3 Stunden bei Raumtemperatur. Das wiederum auf 0° gekühlte Reaktionsgemisch wird hierauf langsam mit 11 ml Wasser und 7,5 ml 2-n. Natronlauge versetzt. Nach weiteren 30 Minuten Rühren filtriert man es über Diatomeenerde. Das Filtergut wäscht man mit Tetrahydrofuran nach und dampft die vereinigten Filtrate im Rotationsverdampfer ein. Der Rückstand wird zweimal aus Aether/Hexan umkristallisiert, wobei man das 2,3-Dimethyl-7-benzofuranmethanol als weisse Kristalle vom Smp. 65,5-66,5° erhält.

e) 17,6 g (0,1 Mol) 2,3-Dimethyl-7-benzofuranmethanol werden in 200 ml Toluol gelöst und unter Rühren mit 18 g Mangandioxid versetzt. Die schwarze Suspension rührt man 7 Tage bei Raumtemperatur und gibt jeden Tag weitere 5 g Mangandioxid zu. Danach wird das Mangandioxid abfiltriert und die Toluollösung im Rotationsverdampfer eingedampft, wobei der 2,3-Dimethyl-7-benzofurancarboxaldehyd als schwach gelbliche Kristalle vom Smp. 63-65° erhalten wird.

f) bis i) Analog Beispiel 4a) bis d) erhält man unter Verwendung der jeweils gleichen Mengen der entsprechenden isomeren Ausgangsstoffe die nachstehend angegebenen Zwischenprodukte, nämlich

f) 2-(2,3-Dimethyl-7-benzofuranyl)-4-hydroxy-4-methyl-6-oxo-1,3-cyclohexandicarbonsäure-diäthylester vom Smp.

130-132°;

g) 3-(2,3-Dimethyl-7-benzofuranyl)-glutarsäure vom Smp. 193-195°;

h) 3-(2,3-Dimethyl-7-benzofuranyl)-glutarsäureanhydrid vom Smp. 143-144°; und

i) 3-(2,3-Dimethyl-7-benzofuranyl)-glutarimid vom Smp. 209-211° (aus Methanol-Aether).

**Beispiel 7**

Analog Beispiel 4 erhält man unter Verwendung von 2,71 g (0,010 Mol) N-Methyl-(2,3-dimethyl-6-benzofuranyl)-glutarimid das 1-Methyl-4-(2,3-dimethyl-6-benzofuranyl)-piperidin. Die rohe Base wird in ca. der 10-fachen Menge (Gewicht/Volumen) eines Gemisches von Methanol-Aether 1:4 gelöst und die Lösung durch Zugabe einer ätherischen Maleinsäurelösung auf einen pH-Wert von etwa 3 eingestellt, wobei das saure 1-Methyl-4-(2,3-dimethyl-6-benzofuranyl)-piperidin-maleinat(1:1) sofort auskristallisiert, Smp. 149-150°. Der Ausgangsstoff wird wie folgt hergestellt:

a) 3,87 g (0,015 Mol) 3-(2,3-Dimethyl-6-benzofuranyl)-glutarsäureanhydrid [vgl. Beispiel 4a) bis e)] löst man in 12,5 ml Toluol und 12,5 ml Eisessig. Zu dieser Lösung gibt man bei Raumtemperatur 10 ml einer 1,88-molaren Lösung von Methylamin in Toluol und erhitzt das Reaktionsgemisch 6 ½ Stunden am Wasserabscheider zum Sieden. Das gekühlte Reaktionsgemisch giesst man auf Eis-Wasser-Gemisch, extrahiert mit Aether und wäscht die organische Phase mit Natriumbicarbonat und gesättigter Natriumchloridlösung neutral. Dann trocknet man die organische Phase über Natriumsulfat und dampft sie im Rotationsverdampfer ein. Das so als sandfarbene Kristalle erhaltene N-Methyl-3-(2,3-dimethyl-6-benzofuranyl)-glutarimid kristallisiert spontan, Smp. 182-185°.

Beispiel 8

Eine Lösung von 2,57 g (0,010 Mol) 1-Formyl-4-(2,3-dimethyl-6-benzofuranyl)-piperidin in 15 ml Tetrahydrofuran wird innerhalb 10 Minuten zu einer mit Eis-Wasser-Gemisch gekühlten Suspension von 0,456 (0,012 Mol) Lithiumaluminiumhydrid in 15 ml abs. Aether getropft, wobei eine exotherme Reaktion eintritt. Das Reaktionsgemisch wird unter Eiskühlung mit 2,5 ml 1-n. Natronlauge versetzt, und noch weitere 30 Minuten im Eis-Wasser-Bad gerührt. Die weisse Suspension wird genutscht, der Filterrückstand mit Aethylacetat nachgewaschen und das Filtrat im Rotationsverdampfer eingedampft, wobei das rohe 1-Methyl-4-(2,3-dimethyl-6-benzofuranyl)-piperidin als gelbliches Oel erhalten wird. Das daraus analog Beispiel 7 bereitete saure Maleinat (1:1) schmilzt bei 149-150°.

Der Ausgangsstoff kann wie folgt hergestellt werden:

a) 2,29 g (0,10 Mol) 4-(2,3-Dimethyl-6-benzofuranyl)-piperidin (vgl. Beispiel 3) werden in 23 ml Ameisensäureäthylester 2 Stunden unter Rückfluss gekocht. Danach dampft man die klare, bräunliche Reaktionslösung im Rotationsverdampfer ein, wobei das 1-Formyl-4-(2,3-dimethyl-6-benzofuranyl)-piperidin als sandfarbene Kristalle vom Smp. 86-90° zurückbleibt. Eine Aether/Hexan umkristallisierte Probe schmilzt bei 89-91°, doch ist das beim Eindampfen erhaltene Produkt genügend rein für die anschliessende Reduktion.

Beispiel 9

Zu einer Suspension von 7,6 g (0,2 Mol) Lithiumaluminiumhydrid in 250 ml Tetrahydrofuran wird innerhalb einer Stunde eine Lösung von 28,3 g (0,1 Mol) 3-(6,7,8,9-Tetrahydro-2-dibenzofuranyl)-glutarsäureimid in 250 ml Tetrahydrofuran bei 0° unter Eis-Wasser-Kühlung zugetropft. Das Reaktionsgemisch wird anschliessend bei Raumtemperatur 3 Stunden weitergerührt und dann wieder auf 0° gekühlt. Bei dieser Temperatur werden zuerst 7,6 ml Wasser, dann

7,6 ml 2-n. Natronlauge und anschliessend 22,8 ml Wasser zugetropft. Die entstandene Suspension wird 30 Minuten bei Raumtemperatur gerührt und dann über Diatomeenerde filtriert. Das Filtrat wird über Natriumsulfat getrocknet und im Wasserstrahlvakuum zur Trockne eingedampft, wobei das rohe 4-(6,7,8,9-Tetrahydro-2-dibenzofuranyl)-piperidin als amorpher gelber Schaum erhalten wird. Durch Umsetzung der Base in Methanol mit einer Lösung von Chlorwasserstoff in Aether erhält man das entsprechende Hydrochlorid in Form weisser Kristalle vom Smp. 260-262°.

In analoger Weise erhält man unter Verwendung von 28,3 g (0,1 Mol) 3-(6,7,8,9-Tetrahydro-1-dibenzofuranyl)-glutarimid das rohe 4-(6,7,8,9-Tetrahydro-1-dibenzofuranyl)-piperidin und daraus das entsprechende Hydrochlorid vom Smp. 291-293° (aus Methanol-Aether).

Die Ausgangsstoffe können wie folgt hergestellt werden:

a) 166,2 g (1 Mol) p-Hydroxybenzoesäure-äthylester und 132,6 g (1 Mol) 2-Chlorcyclohexanon werden in 1500 ml trockenem Aceton gelöst und mit 415 g (3 Mol) wasserfreiem Kaliumcarbonat versetzt. Das Gemisch wird 20 Stunden unter Rückfluss gekocht und dann auf Raumtemperatur abgekühlt. Die entstandene Suspension wird filtriert und das Filtrat im Wasserstrahlvakuum eingedampft. Der ölige Rückstand wird in 500 ml Acethylacetat gelöst, die organische Lösung zweimal mit je 200 ml kalter 1-n. Natronlauge und einmal mit 250 ml Eiswasser gewaschen, über Natriumsulfat getrocknet und im Wasserstrahlvakuum zur Trockne eingedampft. Das erhaltene gelbe Oel wird im Hoch-vakuum destilliert, wobei man den p-(2-Oxocyclohexyloxy)-benzoesäure-äthylester vom Kp. 158-164°/0,1 Torr erhält. Dieser Ester kann aus Aether/Hexan kristallisiert werden, Smp. 66-68°.

In analoger Weise erhält man unter Verwendung von 166,2 g (1 Mol) m-Hydroxybenzoesäure-äthylester den m-(2-Oxocyclohexyloxy)-benzoesäure-äthylester vom Kp.

140-160°/0,08 Torr, Smp. 65-66°.

b) 262,3 g (1 Mol) p-(2-Oxocyclohexyloxy)-benzoesäure-äthylester werden auf 10° gekühlt und tropfenweise innerhalb etwa 2 Stunden mit 250 ml konzentrierter Schwefelsäure versetzt. Die Temperatur wird dabei mit einem Eis-Wasser-Bad zwischen 10° und 20° gehalten. Nach beendeter Zugabe wird das Gemisch noch 3 Stunden bei Raumtemperatur gerührt und anschliessend auf ein 2:1 Gemisch von gesättigter wässriger Natriumcarbonatlösung und Eis gegossen. Die wässrige Phase wird einmal mit etwa 1000 ml Aether und dann noch dreimal mit je 300 ml Aether extrahiert, die vereinigten organischen Phasen werden viermal mit je 300 ml 2-n. Natronlauge gewaschen, über Natriumsulfat getrocknet und im Wasserstrahlvakuum eingedampft. Der ölige Rückstand wird durch Filtration über eine Schicht Silicagel gereinigt, wobei als Lösungs- und Eluiermittel Chloroform verwendet wird. Nach Abdampfen des Chloroforms erhält man den 6,7,8,9-Tetrahydro-2-dibenzofurancarbonsäure-äthylester als gelbliches Oel.

Die nach der Extraktion mit Aether zurückgebliebene alkalische, wässrige Phase wird mit konzentrierter Salzsäure sauer gestellt. Die ausgefallene Substanz wird abfiltriert, mit Wasser gut gewaschen und im Hochvakuum getrocknet, wobei man amorphe 6,7,8,9-Tetrahydro-2-dibenzofurancarbonsäure erhält.

Analog erhält man ausgehend von 262,3 g (1 Mol) m-(2-Oxocyclohexyloxy)-benzoesäure-äthylester den öligen 6,7,8,9-Tetrahydro-1-dibenzofurancarbonsäure-äthylester zusammen mit der freien 6,7,8,9-Tetrahydro-1-dibenzofurancarbonsäure vom Smp. 150-153°.

$c^1$) 122 g (0,5 Mol) 6,7,8,9-Tetrahydro-2-dibenzofurancarbonsäure-äthylester werden in 300 ml Tetrahydrofuran gelöst und zu einer auf 0° gekühlten Suspension von 20 g (0,52 Mol) Lithiumaluminiumhydrid in 300 ml Tetrahydrofuran innerhalb etwa einer Stunde zugetropft. Das Gemisch

wird anschliessend eine Stunde bei 0° und 3 Stunden bei Raumtemperatur gerührt und dann wieder auf 0° gekühlt. Bei dieser Temperatur werden tropfenweise zuerst 20 ml Wasser, dann 20 ml 2-n. Natronlauge und anschliessend 60 ml Wasser zugegeben. Die gebildete Suspension wird 30 Minuten bei Raumtemperatur gerührt und dann über Diatomeenerde filtriert. Der Filterrückstand wird mit Aether gewaschen und die vereinigten Filtrate werden über Natriumsulfat getrocknet und im Wasserstrahlvakuum eingedampft, wobei das 6,7,8,9-Tetrahydro-2-dibenzofuranmethanol als gelbliches Oel zurückbleibt. Es kann aus Aethylacetat-Toluol kristallisiert werden und schmilzt dann bei 63-65°.

$c^2$) Nach demselben Verfahren erhält man aus 0,5 Mol (108 g) 6,7,8,9-Tetrahydro-2-dibenzofurancarbonsäure ebenfalls das 6,7,8,9-Tetrahydro-2-dibenzofuranmethanol.

In analoger Weise erhält man unter Verwendung gleicher Mengen 6,7,8,9-Tetrahydro-1-dibenzofurancarbonsäureäthylester bzw. freier 6,7,8,9-Tetrahydro-1-dibenzofurancarbonsäure das 6,7,8,9-Tetrahydro-1-dibenzofuranmethanol als gelbliche Kristalle vom Smp. 43-45°.

d) 40,4 g (0,2 Mol) 6,7,8,9-Tetrahydro-2-dibenzofuranmethanol werden in 800 ml Toluol gelöst und mit 176 g (2 Mol) Mangandioxid versetzt. Die dunkle Suspension wird 72 Stunden bei Raumtemperatur gerührt, dann über Diatomeenerde filtriert und das erhaltene Filtrat im Wasserstrahlvakuum zur Trockne eingedampft. Man erhält so den 6,7,8,9-Tetrahydro-2-dibenzofurancarboxaldehyd als dunkelgelbes, dünnschichtchromatographisch (Toluol/Aethylacetat 1:1) einheitliches öliges Produkt, das ohne zusätzliche Reinigung für weitere Umsetzungen verwendet werden kann.

In analoger Weise erhält man ausgehend von 40,4 g (0,2 Mol) 6,7,8,9-Tetrahydro-1-dibenzofuranmethanol den 6,7,8,9-Tetrahydro-1-dibenzofurancarboxaldehyd

als gelbliche Kristalle vom Smp. 55-57°.
e) Eine Lösung von 40,0 g (0,2 Mol) 6,7,8,9-Tetrahydro-2-dibenzofurancarboxaldehyd in 500 ml absolutem Aethanol wird mit 52,0 g (0,4 Mol) Acetessigsäure-äthylester und 6 ml Piperidin versetzt. Beim Rühren bei Raumtemperatur bildet sich allmählich ein kristalliner Niederschlag. Dieser wird nach 72 Stunden Rühren abfiltriert, mit Hexan gewaschen und im Hochvakuum getrocknet, wobei der 2-(6,7,8,9-Tetrahydro-2-dibenzofuranyl)-4-hydroxy-4-methyl-6-oxo-1,3-cyclohexandicarbonsäure-diäthylester als gelbliche Kristalle vom Smp. 168-170° erhalten wird. Eine aus Aethanol umkristallisierte Probe schmilzt bei 171-173°.

Analog erhält man unter Verwendung von 40,0 g (0,2 Mol) 6,7,8,9-Tetrahydro-1-dibenzofurancarboxaldehyd den 2-(6,7,8,9-Tetrahydro-1-dibenzofuranyl)-4-hydroxy-4-methyl-6-oxo-1,3-cyclohexandicarbonsäure-diäthylester als blass gelbe Kristalle vom Smp. 155-157°.
f) Zu einer Lösung von 120 g (3 Mol) Natriumhydroxid in 125 ml Wasser mit 250 ml Aethanol lässt man innerhalb 30 Minuten bei 90° eine Lösung von 44,2 g (0,1 Mol) 2-(6,7,8,9-Tetrahydro-2-dibenzofuranyl)-4-hydroxy-4-methyl-1,3-cyclohexandicarbonsäure-diäthylester in 250 ml Dioxan zutropfen. Das Reaktionsgemisch wird 30 Minuten auf 90° erhitzt und dann durch Abdestillieren der Lösungsmittel auf ca. 1/3 seines Volumens eingeengt. Nach dem Verdünnen mit 750 ml Wasser werden unter Eis-Wasser-Kühlung 350 ml konzentrierte Salzsäure zugegeben. Die ausgefallene rohe Säure wird abfiltriert, mit Wasser gut gewaschen und getrocknet. Man erhält so die 3-(6,7,8,9-Tetrahydro-2-dibenzofuranyl)-glutarsäure als blass gelbbraune Kristalle vom Smp. 165-170°. Eine aus Aethanol-Wasser kristallisierte Probe schmilzt bei 169-174°.

Analog erhält man unter Verwendung von 44,2 g

(0,1 Mol) des zweiten gemäss e) erhaltenen Diäthylesters die 3-(6,7,8,9-Tetrahydro-1-dibenzofuranyl)-glutarsäure als gelbliche Kristalle vom Smp. 176-181°
g) 30,2 g (0,1 Mol) 3-(6,7,8,9-Tetrahydro-2-dibenzofuranyl)-glutarsäure werden mit 100 ml Acetanhydrid 1 ½ Stunden unter Rückfluss gekocht. Beim Stehenlassen der abgekühlten dunklen Lösung bildet sich ein kristalliner Niederschlag, der nach 48 Stunden abfiltriert und mit kaltem Toluol gewaschen wird. Das so erhaltene 3-(6,7,8,9-Tetrahydro-2-dibenzofuranyl)-glutarsäureanhydrid schmilzt bei 164-166°. Aus dem Filtrat erhält man nach dem Einengen im Wasserstrahlvakuum eine zusätzliche Menge 3-(6,7,8,9-Tetrahydro-2-dibenzofuranyl)-glutarsäureanhydrid als gelbliche Kristalle vom gleichen Smp.

In analoger Weise erhält man unter Verwendung von 30,2 g (0,1 Mol) 3-(6,7,8,9-Tetrahydro-1-dibenzofuranyl)-glutarsäure das 3-(6,7,8,9-Tetrahydro-1-dibenzofuranyl)-glutarsäureanhydrid als gelbe Kristalle vom Smp. 139-140°.
h[1]) 28,4 g (0,1 Mol) 3-(6,7,8,9-Tetrahydro-2-dibenzofuranyl-2-glutarsäureanhydrid werden portionenweise bei 40° zu 100 ml 25 %-ige wässrige Ammoniaklösung gegeben. Das Gemisch wird eine Stunde auf 70° erhitzt und dann im Wasserstrahlvakuum zur Trockne eingedampft. Der gelbe Rückstand wird anschliessend 3 Stunden auf 150° erhitzt. Das erhaltene graubraune Produkt wird in 300 ml Dioxan gelöst und durch Zugabe von Aether zur Kristallisation gebracht, wobei man das 3-(6,7,8,9-Tetrahydro-2-dibenzofuranyl)-glutarsäureimid als blass gelbbraune Kristalle vom Smp. 184-193° erhält.

Das obige Imid kann ebensogut auch analog h[2]) hergestellt werden.
h[2]) 28,4 g (0,1 Mol) 3-(6,7,8,9-Tetrahydro-1-dibenzofuranyl)-glutarsäureanhydrid werden zusammen mit 77 g Ammoniumacetat (1 Mol) auf 150° erhitzt. Zur klaren, dunklen

Schmelze werden nach 2 bzw. 6 Stunden nochmals je 20 g Ammoniumacetat zugegeben. Nach 8 Stunden wird die Schmelze auf 1 Liter Eis-Wasser-Gemisch gegossen. Das dabei ausgefallene 3-(6,7,8,9-Tetrahydro-1-dibenzofuranyl)-glutarsäureimid wird abfiltriert, getrocknet und aus Dioxan-Aether umkristallisiert, worauf man es als graue Kristalle vom Smp. 229-231° erhält.

Das obige Imid kann auch analog $h^1$) hergestellt werden.

Beispiel 10

15,0 g (0,06 Mol) 1-Methyl-4-[2-methyl-5-benzofuranyl)-4-piperidinol werden in 250 ml Hexamethylphosphorsäuretriamid gelöst und die Lösung unter einer inerten Stickstoffatmosphäre eine Stunde auf 230° erhitzt. Nach dem Abkühlen wird das Lösungsmittel im Wasserstrahlvakuum bei 110° abdestilliert, der dunkelbraune, ölige Rückstand in Toluol gelöst und die organische Phase mit einer ca. 10 %-igen wässrigen Lösung von Methansulfonsäure extrahiert. Der saure Extrakt wird zweimal mit Toluol gewaschen, dann in der Kälte mit konzentrierter Natronlauge auf pH 10 gestellt und mit Chloroform extrahiert. Die organische Phase wird mit Wasser gewaschen, über Natriumsulfat getrocknet und im Wasserstrahlvakuum zur Trockne eingedampft. Der ölige Rückstand wird im Hochvakuum destilliert, wobei man das 1-Methyl-4-(2-methyl-5-benzofuranyl)-1,2,3,6-tetrahydropyridin vom Kp. 115-128°/0,03 Torr., Smp. 59-62°, erhält. Das mit Chlorwasserstoff in Aethylacetat bereitete Hydrochlorid schmilzt bei 195-197°.

Der Ausgangsstoff wird wie folgt hergestellt:

a) 63,3 g (0,3 Mol) 2-Methyl-5-brom-benzofuran werden in 200 ml trockenem Aether gelöst, im Eis-Natriumchlorid-Bad auf -10° gekühlt und mit 200 ml einer ca.

2-molaren Lösung von Butyl-lithium in Hexan tropfenweise versetzt. Dabei lässt man die Temperatur nicht über 0° steigen. Das Reaktionsgemisch wird während einer Stunde bei dieser Temperatur gehalten, und hierauf tropfenweise mit einer Lösung von 34 g (0,3 Mol) 1-Methyl-4-piperidinon in 100 ml trockenem Aether versetzt. Die Innentemperatur wird zwischen 0° und + 10° gehalten. Das Reaktionsgemisch wird anschliessend bei Raumtemperatur während 15 Stunden gerührt und dann auf ca. 300 ml Eis-Wasser-Gemisch gegossen. Die organische Phase wird abgetrennt und die wässrige Phase zweimal mit je 100 ml Aethylacetat extrahiert. Die organischen Phase werden vereinigt, mit Wasser gewaschen und hierauf zweimal mit einer ca. 10 %-igen wässrigen Lösung von Methansulfonsäure extrahiert. Die vereinigten sauren Lösungen werden über Diatomeenerde filtriert, mit Eis-Wasser-Gemisch gekühlt, mit konzentrierter Natronlauge alkalisch gestellt und mit Chloroform dreimal extrahiert. Die organischen Phasen werden vereinigt, über Natriumsulfat getrocknet und im Wasserstrahlvakuum zur Trockne eingedampft. Das erhaltene Rohprodukt wird aus 2000 ml Hexan kristallisiert, wobei das 1-Methyl-4-(2-methyl-5-benzofuranyl)-4-piperidinol vom Smp. 118-120° erhalten wird. Das aus der freien Base mit Chlorwasserstoff hergestellte Hydrochlorid kristallisiert aus Aceton, Smp. 188-191°.

Beispiel 11

10,8 g 1-Methyl-4-(2-methyl-5-benzofuranyl)-1,2,3,6-tetrahydropyridin (vgl. Beispiel 10) in 100 ml Methanol werden in Gegenwart von 1,0 g Palladium-Kohle-Katalysator (5% Pd) bei Raumtemperatur und Normaldruck bis zur Aufnahme der äquimolaren Menge Wasserstoff hydriert. Dann wird der Katalysator abfiltriert, das Methanol abgedampft und der Rück-

stand im Hochvakuum destilliert, wobei man das 1-Methyl-4-(2-methyl-5-benzofuranyl)-piperidin als Oel vom Kp. 112-114°/0,01 Torr erhält. Das in üblicher Weise in Aethylacetat bereitete Hydrochlorid schmilzt zwischen 220° und 235°.

Beispiel 12

7,3 g (0,03 Mol) 1-Methyl-4-(2-methyl-5-benzofuranyl)-piperidin (vgl. Beispiel 11) werden zusammen mit 11,0 g (0,1 Mol) Chlorameisensäureäthylester in 125 ml Toluol 15 Stunden bei 50-55° gerührt. Das Lösungsmittel und der überschüssige Chlorameisensäureäthylester werden im Wasserstrahlvakuum bei 70° Badtemperatur abdestilliert, der erhaltene Rückstand in Toluol gelöst und die Toluollösung mit Wasser, 2-n. Natronlauge, Wasser, 2-n. Salzsäure und wiederum Wasser gewaschen. Dann wird die Toluollösung über Natriumsulfat getrocknet und im Wasserstrahlvakuum zur Trockne eingedampft. Der als Oel zurückbleibende, rohe 4-(2-Methyl-2-benzofuranyl)-1-piperidincarbonsäure-äthylester kann direkt weiterverwendet werden. 9,1 g (0,03 Mol) des obigen rohen Esters und 15 g festes Kaliumhydroxid werden in 55 ml Diäthylenglykol und 10 ml Wasser gelöst und 15 Stunden in einer Stickstoffatmosphäre unter Rückfluss gekocht. Nach dem Abkühlen wird das Reaktionsgemisch mit 250 ml Toluol verdünnt, die organische Phase abgetrennt, mit Wasser gewaschen und anschliessend mit 10%-iger wässriger Methansulfonsäurelösung extrahiert. Der saure Extrakt wird mit Toluol gewaschen, mit konzentrierter Natronlauge in der Kälte stark alkalisch gestellt und mit Chloroform extrahiert. Die Chloroformlösung wird mit Wasser gewaschen, über Natriumsulfat getrocknet und im Wasserstrahlvakuum eingedampft. Das erhaltene Oel wird im Hochvakuum destilliert und liefert

das 4-(2-Methyl-5-benzofuranyl)-piperidin vom Kp. 108-111°/ 0,015 Torr.

In üblicher Weise wird daraus das Hydrochlorid vom Smp. 212-124° erhalten.

Beispiel 13

2,8 g (0,013 Mol) 4-(2-Methyl-5-benzofuranyl)-piperidin (vgl. Beispiel12) und 2,4 g (0,02 Mol) 2-Propinylbromid werden in einer Suspension von 10 g Kaliumcarbonat in 120 ml Methanol gelöst und bei Raumtemperatur gerührt. Nach 24 Stunden werden nochmals 0,24 g 2-Propinylbromid hinzugefügt und das Reaktionsgemisch wird weitere 24 Stunden bei Raumtemperatur gerührt. Hierauf wird es filtriert, der Filterrückstand mit Aethylacetat nachgewaschen und die vereinigten Filtrate im Wasserstrahlvakuum zur Trockne eingedampft. Der Rückstand wird wieder in Aethylacetat gelöst, die Lösung dreimal mit Wasser gewaschen, über Natriumsulfat getrocknet und im Wasserstrahlvakuum zur Trockne eingedampft. Der erhaltene Rückstand wird an 200 g Aluminiumoxid chromatographiert (Eluiermittel Methylenchlorid), wobei nach Abdampfen des Lösungsmittels kristallines 1-(2-Propinyl)-4-(2-methyl-5-benzofuranyl)-piperidin erhalten wird. Das aus der Base in üblicher Weise bereitete Hydrochlorid schmilzt bei 185-187°.

Beispiel 14

Zu einer Lösung von 2,29 g (0,010 Mol) 4-(2,3-Dimethyl-5-benzofuranyl)-piperidin (vgl. Beispiel 1) in 25 ml Aceton gibt man 4,15 g (0,030 Mol) wasserfreies Kaliumcarbonat und tropft innerhalb 10 Minuten unter Rühren eine Lösung von 1,3 g (0,011 Mol) 2-Propinylbromid in 11 ml Aceton zu. Das Reaktionsgemisch erhitzt man 4 Stunden auf 40°, kühlt es dann auf Raumtemperatur und filtriert die anorganischen

Salze ab. Das Filtrat dampft man im Rotationsverdampfer ein. Das zurückbleibende braune Oel zeigt im Dünnschichtchromatogramm (Silicagel/5 % Methanol/Chloroform) einen Hauptfleck bei Rf ca. 0,7 neben 4 Nebenflecken. Das erhaltene Oel wird an 50 g Kieselgel unter Verwendung von Chloroform als Lösungs- und Eluiermittel chromatographiert, wobei als Hauptfraktion das 1-(2-Propinyl)-4-(2,3-dimethyl-5-benzofuranyl)-piperidin als gelbliches Oel, Rf ca. 0,7 erhalten wird. Diese Base wird in ca. 3 ml Methanol gelöst und durch Ansäuern mit Chlorwasserstofflösung ins Hydrochlorid übergeführt, welches durch Zugabe von ca. 20 ml Aether zur Kristallisation gebracht und abgenutscht wird. Das so als weisse Kristalle erhaltene Hydrochlorid schmilzt bei 224-225°.

Beispiel 15

Analog Beispiel 8 erhält man durch Reduktion mittels Lithiumaluminiumhydrid

aus 2,57 g (0,010 Mol) 1-Formyl-4-(2,3-dimethyl-4-benzofuranyl)-piperidin das 1-Methyl-4-(2,3-dimethyl-4-benzofuranyl)-piperidin und dessen (1:1)-Maleinat,

aus 2,57 g (0,010 Mol) 1-Formyl-4-(2,3-dimethyl-7-benzofuranyl)-piperidin das 1-Methyl-4-(2,3-dimethyl-7-benzofuranyl)-piperidin und dessen (1:1)-Maleinat,

aus 2,83 g (0,010 Mol) 1-Formyl-4-(6,7,8,9-tetrahydro-2-dibenzofuranyl)-piperidin das 1-Methyl-4-(6,7,8,9-tetrahydro-2-dibenzofuranyl)-piperidin und dessen (1:1)-Maleinat, und

aus 2,83 g (0,010 Mol) 1-Formyl-4-(6,7,8,9-tetrahydro-1-dibenzofuranyl)-piperidin das 1-Methyl-4-(6,7,8,9-tetrahydro-1-dibenzofuranyl)-piperidin und dessen (1:1)-Maleinat.

Anstelle der (1:1)-Maleinate kann man z.B. auch in üblicher Weise die Hydrochloride der obigen Basen herstellen.

Die als Ausgangsstoffe benötigten 1-Formylverbindungen werden aus den entsprechenden, in den Beispielen 5, 6 bzw. 9 beschriebenen Verbindungen ohne 1-Substituent analog Beispiel 8a) hergestellt.

Beispiel 16

Analog Beispiel 14 erhält man

unter Verwendung von 2,29 g (0,010 Mol) 4-(2,3-Dimethyl-6-benzofuranyl)-piperidin (vgl. Beispiel 3) das 1-(2-Propinyl)-(2,3-dimethyl-6-benzofuranyl)-piperidin und dessen Hydrochlorid,

unter Verwendung von 2,29 g (0,010 Mol) 4-(2,3-Dimethyl-4-benzofuranyl)-piperidin (vgl. Beispiel 5) das 1-(2-Propinyl)-2,3-dimethyl-4-benzofuranyl)-piperidin und dessen Hydrochlorid,

unter Verwendung von 2,29 g (0,010 Mol) 4-(2,3-Dimethyl-7-benzofuranyl)-piperidin (vgl. Beispiel 6) das 1-(2-Propinyl)-4-(2,3-dimethyl-7-benzofuranyl)-piperidin und dessen Hydrochlorid,

unter Verwendung von 2,55 g (0,010 Mol) 4-(6,7,8,9-Tetrahydro-2-dibenzofuranyl)piperidin (vgl. Beispiel 9) das 1-(2-Propinyl)-4-(6,7,8,9-tetrahydro-2-dibenzofuranyl)-piperidin und dessen Hydrochlorid, und

unter Verwendung 2,55 g (0,010 Mol) 4-(6,7,8,9-Tetrahydro-1-dibenzofuranyl)-piperidin (vgl. Beispiel 9) das 1-(2-Propinyl)-4-(6,7,8,9-tetrahydro-1-dibenzofuranyl)-piperidin und dessen Hydrochlorid.

Beispiel 17

Analog Beispiel 4 werden 14,4 (0,050 Mol) 3-(2,3-Dimethyl-7-methoxy-5-benzofuranyl)-glutarimid in eine Suspension von 3,8 g (0,10 Mol) Lithiumaluminiumhydrid in 500 ml Aether reduziert und die erhaltene Base vom Smp. 75-77° in das 4-(2,3-Dimethyl-7-methoxy-5-benzofuranyl)-piperidin-hydrochlorid vom Smp. 277-277,5° übergeführt.

Der Ausgangsstoff wird wie folgt hergestellt:

a) 196.2 g (1,0 Mol) 4-Hydroxy-3-methoxy-benzoesäure-äthylester (Vanillinsäureäthylester) und 106,5 g (1,0 Mol) 3-Chlor-2-butanon werden in 1100 ml trockenem Aceton gelöst und mit 415 g (3,0 Mol) wasserfreiem Kalium carbonat versetzt. Das Gemisch wird 24 Stunden unter Rückfluss gekocht und dann auf Raumtemperatur abgekühlt. Die entstandene Suspension wird filtriert und das Filtrat im Wasserstrahlvakuum eingeengt. Der ölige Rückstand wird direkt im Hochvakuum destilliert, wobei man den 3-Methoxy-4-(1-methyl-2-oxopropoxy)-benzoesäure-äthylester vom Kp. 132-134° (0,1 Torr) als viskoses, schwach gelbliches Oel erhält.

b) 214 g (0,8 Mol) 3-Methoxy-4-(1-methyl-2-oxopropoxy)-benzoesäure-äthylester werden bei Raumtemperatur mit 1600 ml 70 %-iger Schwefelsäure versetzt. Das Reaktionsgemisch verfärbt sich dabei nach dunkelrot-braun. Danach erwärmt man das Reaktionsgemisch 4 Stunden auf 60°. Dabei entsteht allmählich ein brauner kristalliner Brei. Das Reaktionsgemisch lässt man auf Raumtemperatur erkalten, giesst es auf 2 kg Eis und extrahiert mit Aethylacetat. Der Extrakt wird zweimal mit Wasser gewaschen, hierauf 5-mal mit je 500 ml 0,5n-Natriumbicarbonatlösung und 3-mal mit je 500 ml 2-n.Natriumcarbonatlösung extrahiert, über Magnesiumfulfat getrocknet und im Wasserstrahlvakuum vollständig eingedampft. Durch Chromatographie an 1,5 kg

Kieselgel mit Chloroform, das 2 % Methanol enthält, erhält man den 2,3-Dimethyl-7-methoxy-5-benzofurancarbonsäure-äthylester, der nach Umkristallisation aus warmem Hexan bei 67-68° schmilzt.

Die weiter oben erhaltenen Natriumcarbonatextrakte werden vereinigt, mit 5-n.-Salzsäure sauer gestellt (pH 2-3) und hierauf mit Aethylacetat extrahiert.

Die so erhaltene neue Aethylacetatphase wird ebenfalls über Magnesiumsulfat getrocknet und anschliessend im Wasserstrahlvakuum eingedampft. Durch Umkristallisation des Rückstandes aus Aethylacetat erhält man die 2,3-Dimethyl-7-methoxy-5-benzofurancarbonsäure vom Smp. 229-231°.

Bei der analogen Aufarbeitung der Natriumbicarbonatextrakte erhält man neben der vorstehend beschriebenen Säure hauptsächlich die durch Verseifung der Estergruppe des Ausgangsmaterials entstehende Säure neben wenig Vanillinsäure.

c) 49,6 g (0,2 Mol) 2,3-Dimethyl-7-methoxy-5-benzofurancarbonsäure-äthylester [oder 44,1 g (0,20 Mol) der entsprechenden freien Säure] werden in einer Suspension von 7,8 g (0,2 Mol) Lithiumaluminiumhydrid in 300 ml wasserfreien Tetrahydrofuran analog Beispiel 1a) zum 2,3-Dimethyl-7-methoxy-5-benzofuranmethanol vom Smp. 83-84° reduziert.

b) 61,8 g (0,3 Mol) 2,3-Dimethyl-7-methoxy-5-benzofuranmethanol werden analog Beispiel 1b) mit 135 g Mangandioxid in 500 ml Toluol zum 2,3-Dimethyl-7-methoxy-5-benzofurancarboxaldehyd vom Smp. 80-81° oxidiert.

c) Analog Beispiel 4a) werden 61,2 g (0,3 Mol) 2,3-Dimethyl-7-methoxy-5-benzofurancarboxaldehyd mit 78,9 g (0,606 Mol) Acetessigsäureäthylester in Gegenwart von 5,4 ml Piperidin in 150 ml Aethanol zum 2-(2,3-Dimethyl-7-methoxy-5-benzofuranyl-4-hydroxy-4-methyl-6-oxo-1,3-cyclohexandicarbonsäure-diäthylester vom Smp. 157-158° kondensiert.

d) 67 g (0,15 Mol) des Reaktionsproduktes von c) werden analog Beispiel 4 b) durch Erwärmen mit 120 g (3 Mol) Natriumhydroxid in 360 ml Wasser und 150 ml Aethanol in die 3-(2,3-Dimethyl-7-methoxy-2-benzofuranyl)-glutarsäure von Smp. 168-169° übergeführt.

e) Analog Beispiel 4c ) erhält man durch Kochen von 45,9 g (0,15 Mol) der nach d) erhaltenen Dicarbonsäure in 180 ml Acetanhydrid das 3-(2,3-Dimethyl-7-methoxy-5-benzofuranyl)-glutarsäureanhydrid vom Smp. 194-195°

f) Durch Umsetzung von 28,8 g (0,1 Mol) des gemäss b) erhaltenen Anhydrids mit 77 g (4 Mol) Ammoniumacetat erhält man analog Beispiel 4 d) das 3-(2,3-Dimethyl-7-methoxy-5-benzofuranyl)-glutarimid vom Smp. 220-222°.

Beispiel 18

Analog Beispiel 9 erhält man durch Reduktion von 31,3 g (0,1 Mol) 3-(4-Methoxy-6,7,8,9-tetrahydro-2-dibenzofuranyl)-glutarsäureimid das 4-(4-Methoxy-6,7,8,9-tetrahydro-2-dibenzofuranyl)-piperidin und dessen Hydrochlorid.

Der Ausgangsstoff wird wie folgt hergestellt:

a) Analog Beispiel 9 a) erhält man unter Verwendung von 196,2 g (1 Mol) 4-Hydroxy-3-methoxybenzoesäure-äthylester den 3-Methoxy-4-(2-oxocyclohexyloxy)-benzoesäure-äthylester.

b) Analog Beispiel 9 b) erhält man ausgehend von 292,3 g (1 Mol) 3-Methoxy-4-(2-oxocyclohexyloxy)-benzoesäure-äthylester den 4-Methoxy-6,7,8,9-tetrahydro-2-dibenzofurancarbonsäure-äthylester

c) Analog Beispiel 9 $c^1$) erhält man durch Reduktion von 137 g (0,5 Mol) 4-Methoxy-6,7,8,9-tetrahydro-2-dibenzofurancarbonsäure-äthylester das 4-Methoxy-6,7,8,9-tetrahydro-2-dibenzofuranmethanol.

d) Analog Beispiel 9 d) erhält man durch Oxidation von 46,4 g (0,2 Mol) 4-Methoxy-6,7,8,9-tetrahydro-2-dibenzofuranmethanol den 4-Methoxy-6,7,8,9-tetrahydro-2-dibenzo-

furancarboxaldehyd.

e) Analog Beispiel 9 e) erhält man unter Verwendung von 46,0 g des nach d) erhalteren Aldehyds den 2-(4-Methoxy-6,7,8,9-tetrahydro-2-dibenzofuranyl)-4-hydroxy-4-methyl-6-oxo-1,3-cyclohexandicarbonsäure-diäthylester.

f) Analog Beispiel 9 f) erhält man ausgehend von 47,2 g (0,1 Mol) des Reaktionsproduktes von e) die 3-(4-Methoxy-6,7,8,9-tetrahydro-2-dibenzofuranyl)-glutarsäure.

g) Analog Beispiel 9 g) erhält man ausgehend von 33,2 g (0,1 Mol) 3-(4-Methoxy-6,7,8,9-tetrahydro-2-dibenzofuranyl)-glutarsäure das 3-(4-Methoxy-6,7,8,9-tetrahydro-2-dibenzofuranyl)-glutarsäureanhydrid.

h) Analog Beispiel 9 $h^2$) erhält man unter Verwendung von 31,4 g (0,1 Mol) des gemäss g) erhaltenen Anhydrids das 3-(4-Methoxy-6,7,8,9-tetrahydro-2-dibenzofuranyl)-glutarsäureimid.

Beispiel 19:

2,97 g (0,010 Mol) N-Cyclopropyl-3-(2,3-dimethyl-6-benzofuranyl)-glutarimid werden analog Beispiel 4 reduziert. Dabei wird das 1-Cyclopropyl-4-(2,3-dimethyl-6-benzofuranyl)-piperidin als farbloses Oel erhalten. Im Dünnschichtchromatogramm auf Kieselgel mit dem Laufmittel Chloroform und 10 % Methanol zeigt das Produkt einen Hauptflecken bei Rf 0.6 neben einem kleinen Flecken am Start und 2 weiteren Nebenflecken mit den Rf-Werten ca. 0,3 resp. 0,8.

a) 2,58 g (0,01 m ) 3-(2,3-Dimethyl-6-benzofuranyl)-glutarsäureanhydrid, (vgl. Beispiel 4 c) 0,855 g entsprechend ca. 1,04 ml Cyclopropylamin (1,5 Moläquivalente) und 11 ml Eisessig werden in 11 ml Toluol gelöst und am Wasserabscheider während 2 Tagen unter Rückfluss gekocht. Danach kühlt man das Reaktionsgemisch auf Raumtemperatur ab, verdünnt es mit 50 ml Aethylacetat und

wäscht es zweimal mit 25 ml Wasser. Hierauf extrahiert man die organische Phase mit 2-n.wässriger Natriumcarbonatlösung zur Entfernung der Säureanteile, wäscht sie dann nochmals mit Wasser und mit gesättigter Natriumchloridlösung (je 25 ml), trocknet sie über Natriumsulfat und dampft das Aethylacetat im Wasserstrahlvakuum ab. Dabei fällt sandfarbenes Rohprodukt vom Smp. 180-182° an. Dieser wird aus warmem Methylenchlorid und Hexan umkristalliert, wobei das N-Cyclopropyl-3-(2,3-dimethyl-6-benzofuranyl)-glutarimid als weisse Blättchen vom Smp. 183-184° erhalten wird.

Beispiel 20

7,4 g (0,02 Mol) 1-Benzyl-4-(2,3,7-trimethyl-5-benzofuranyl)-piperidin-hydrochlorid werden in 150 ml Aethanol gelöst und nach Zugabe von 0,75 g Palladium/Kohle (5 %) unter Normaldruck bei Raumtemperatur während 9 Stunden hydriert. Danach wird der Katalysator über Diatomeenerde abfiltriert und das Filtrat im Wasserstrahlvakuum eingedampft. Das Rohprodukt kristallisiert man aus Aethanol/Aether und erhält das 4-(2,3,7-Trimethyl-5-benzofuranyl)-piperidin-hydrochlorid vom Smp. 307-308°.

Der Ausgangsstoff wird wie folgt hergestellt:

a) Analog Beispiel 17 a) erhält man unter Verwendung von 180,2 g (1,0 Mol) 4-Hydroxy-3-methylbenzoesäure-äthylester den 4-(1-Methyl-2-oxopropoxy)-3-methylbenzoesäure-äthylester vom Kp. 112-113° (0,02 Torr).

b) 250,3 g (1,0 Mol) 4-(1-Methyl-2-oxopropoxy)-3-methylbenzoesäure-äthylester werden in 400 ml 94%-iger Schwefelsäure zunächst 30 Minuten bei Raumtemperatur und anschliessend 30 Minuten bei 60° gerührt. Das Reaktionsgemisch wird auf Raumtemperatur abgekühlt, auf 2 kg Eis gegossen und mit Aethylacetat extrahiert. Die organische Phase wird zweimal mit Wasser gewaschen und hierauf

3-mal mit je 500 ml 2-n.Natriumcarbonatlösung extrahiert. Die Natriumcarbonatlösungen werden mit 5-n. Salzsäure sauer gestellt (pH ca. 2-3) und hierauf mit Aethylacetat extrahiert. Die organische Phase wird über Magnesiumsulfat getrocknet und im Wasserstrahlvakuum vollständig eingedampft. Aus dem Rückstand erhält man durch einmalige Umkristallisation aus heissem Aethanol die 2,3,7-Trimethyl-5-benzofurancarbonsäure vom Smp. 235-237°.

c) 46,4 g (0,2 Mol) 2,3,7-Trimethyl-5-benzofurancarbonsäure-äthylester [oder 40,8 g (0,2 Mol) der entsprechenden freien Säure] werden in einer Suspension von 7,8 g (0,2 Mol) Lithiumaluminiumhydrid in 300 ml wasserfreiem Tetrahydrofuran analog Beispiel 1 a) zum 2,3,7-Trimethyl-5-benzofuranmethanol vom Smp. 85-86° reduziert.

d) 57,0 g (0,3 Mol) 2,3,7-Trimethyl-5-benzofuranmethanol werden analog Beispiel 1 b) mit 135 g Mangandioxid in 500 ml Toluol zum 2,3,7-Trimethyl-5-benzofurancarboxaldehyd vom Smp. 57-58° oxidiert.

e) Analog Beispiel 4 a) werden 56,4 g (0,3 Mol) 2,3,7-Trimethyl-5-benzofurancarboxaldehyd mit 78,9 g (0,606 Mol) Acetessigsäure-äthylester in Gegenwart von 5,4 ml Piperidin in 150 ml Aethanol zum 2-(2,3,7-Trimethyl-5-benzofuranyl-4-hydroxy-4-methyl-6-oxo-1,3-cyclohexandicarbonsäure-diäthylester vom Smp. 168,5-171° kondensiert

f) 64,6 g (0,15 Mol) des Reaktionsproduktes von e) werden analog Beispiel 4 b) durch Erwärmen mit 120 g (3 Mol) Natriumhydroxid in 360 ml Wasser und 150 ml Aethanol in die 3-(2,3,7-Trimethyl-2-benzofuranyl)-glutarsäure vom Smp. 167-169° übergeführt.

g) Analog Beispiel 4 c) erhält man durch Kochen von 43,5 g (0,15 Mol) der nach f) erhaltenen Dicarbonsäure in 180 ml Acetanhydrid das 3-(2,3,7-Trimethyl-5-benzofuranyl)-glutarsäureanhydrid vom Smp. 170°.

a) Analog dem Beispiel 7a, erhält man unter Verwendung von 13,6 g (0,05 m) 3-(2,3,7-Trimethyl-5-benzofuranyl)-glutarsäureanhydrid und 5,8 g (0,055 Mol) Benzylamin in 30 ml Toluol und 50 ml Eisessig das 6-Benzyl-3-(2,3,7-Trimethyl-5-benzofuranyl)-glutarimid vom Smp. 138-138,5°.
b) Analog Beispiel 4 erhält man unter Verwendung von 36.1 g (0,1 Mol) 6-Benzyl-3-(2,3,7-trimethyl-5-benzofuranyl)-glutarimid und 7,6 g (0,9 Mol) Lithiumaluminiumhydrid in 500 ml Aether das 1-Benzyl-4-(2,3,7-Trimethyl-5-benzofuranyl)-piperidin-hydrochlorid vom Smp. 284-286°.

Beispiel 21

24,2 g (0,05 Mol) 3-(2,3-Dimethyl-7-brom-benzofuranyl)-1,5-pentandiyl-dimethansulfonat werden in 700 ml 4,4-n. aethanolischer Ammoniaklösung gelöst und im Druckgefäss 16 Stunden auf 65-70°. erwärmt. Danach lässt man das Reaktionsgemisch erkalten und entfernt überschüssiges Ammoniak und das Aethanol im Wasserstrahlvakuum. Den anfallenden gelben, öligen Rückstand nimmt man in Aethylacetat auf und extrahiert die erhaltene Lösung mit 2-n. Salzsäure. Die saure wässrige Lösung wird durch Diatomeenerde filtriert und nach Kühlung mit Eis- Wasser-Gemisch mit 5n. Natronlauge alkalisch gestellt. Die alkalische Lösung wird mit Aethylacetat extrahiert. Die Aethylacetatextrakte werden über wasserfreiem Natriumsulfat getrocknet und im Wasserstrahlvakuum eingedampft. Die erhaltene rohe Base wird in Methanol gelöst und mit aetherischer Chlorwasserstofflösung ins Hydrochlorid übergeführt. Durch Zugabe von Aether fällt man das rohe Hydrochlorid aus und kristalliert es aus Aethanol/Aether, wobei man das 4-(2,3-Dimethyl-7-brom-5-benzofuranyl)-piperidin-hydrochlorid vom Smp. 304-305° erhält.

Der Ausgangsstoff wird wie folgt hergestellt.
a) Analog Beispiel 17 a) erhält man unter Verwendung von 244,36 g (1,0 Mol) 3-Brom-4-hydroxybenzoesäure-äthylester den 3-Brom-4-( ... )-benzoesäure-äthyl-

ester vom Kp. 142-143° (0,11 Torr)

b) Analog Beispiel 17 b) erhält man unter Verwendung von 315,2 g (1 Mol 3-Brom-4(1-methyl-2-oxopropoxy)-benzoesäureäthylester und 400 g 94 %iger Schwefelsäure zuerst unter Eiskühlung, dann eine Stunde bei Raumtemperatur und anschliessend 8 Stunden bei 50°, den 2,3-Dimethyl-7-brom-5-benzofurancarbonsäure-ethylester vom Smp. 110-111° durch Umkristallisation des Neutralteils aus heissem Aethanol, sowie die 2,3-Dimethyl-7-brom -5-benzofuranyl-carbonsäure vom Smp. 249-251° durch Umkristallisation des Säureteils aus heissem Aethanol.

c) 59,4 g (0,2 Mol) 2,3-Dimethyl-7-brom-5-benzofurancarbonsäure-äthylester [oder 53,8 g (0,2 Mol) der entsprechenden freien Säure] werden in einer Suspension von 7,8 g (0,2 Mol) Lithiumaluminiumhydrid in 300 ml wasserfreiem Tetrahydrofuran analog Beispiel 1 a) zum 2,3-Dimethyl-7-brom-5-benzofuranmethanol von Smp. 131-132° reduziert.

d) 76,5 g (0,3 Mol) 2,3-Dimethyl-7-brom-5-benzofuranmethanol werden analog Beispiel 1 b) mit 135 g Mangandioxid in 650 ml Toluol zum 2,3-Dimethyl-7-brom-5-benzofurancarboxaldehyd vom Smp. 126° oxidiert.

e) Analog Beispiel 4 a) werden 75,9 g (0,3 Mol) 2,3-Dimethyl-7-brom-5-benzofurancarboxaldehyd mit 78,9 g (0,606 Mol) Acetessigsäure-äthylester in Gegenwart von 5,4 ml Piperidin in 200 ml Aethanol zum 2-(2,3-Dimethyl-7-brom-5-benzofuranyl-4-hydroxy-4-methyl-6-oxo-1,3-cyclohexandicarbonsäure-diäthylester vom Smp. 194-195° kondensiert.

f) 74,25 g (0,15 Mol) des Reaktionsproduktes von c) werden analog Beispiel 4 b) durch Erwärmen mit 120 g (3 Mol) Natriumhydroxid in 360 ml Wasser und 150 ml Aethanol in die 3-(2,3-Dimethyl-7-brom-2-benzofuranyl)-glutarsäure vom Smp. 197-198° übergeführt.

g) 53,2 g (0,15 Mol) 3-(2,3-Dimethyl-7-brom-5-benzofuranyl)-glutarsäure werden in 500 ml wasserfreiem Tetrahydrofuran gelöst und unter Kühlung auf 5-10° und Rühren mit 450 ml (0,45 Mol) einer 1,0-molaren Boranlösung in Tetrahydrofuran versetzt. Nach beendeter Zugabe der Boranlösung lässt man das Reaktionsgemisch sich auf Raumtemperatur erwärmen und rührt es bei dieser Temperatur weitere 3 Stunden. Danach kühlt man das Reaktionsgemisch im Eis-Wasser-Bad und tropft 225 ml einer 6-n. Lösung von Salzsäure in Methanol zu, wobei Wasserstoff freigesetzt wird (bei nicht vollständiger Methanolyse des intermediär entstehenden Boresters fällt dieser aus dem anfallenden milchigen Oel aus, sein Schmelpunkt liegt bei 143-146°. (Achtung auf Wasserstoffabgang). Das Reaktionsgemisch lässt man erneut sich auf Raumtemperatur erwärmen und rührt es dann noch eine Stunde. Darauf werden die Lösungsmittel im Wasserstrahlvakuum abgedampft. Das anfallende milchige Oel chromatographiert man zur Abtrennung apolarer Nebenprodukte an Kieselgel mit Methylenchlorid mit 10 % Methanol. Als Hauptfraktion wird das 3-(2,3-Dimethyl-7-brom-5-benzofuranyl-1,5-pentandiol vom Smp. 98-101° eluiert. Nach einmaliger Umkristallisation erhält man reines Diol vom Smp. 102-103°.

h) 16,4 g (0.05 Mol) 3-(2,3-Dimethyl-7-brom-5-benzofuranyl)-1,5-pentandiol werden in 200 ml Pyridin gelöst. Die Lösung wird auf -8 bis -10° gekühlt und bei dieser Temperatur mit 8,5 ml (= 12,6 g, 0,11 Mol) Methansulfonsäurechlorid innerhalb 30 Minuten tropfenweise versetzt. Man lässt bei -8 bis -10° während 1 1/2 Stunden reagieren, giesst dann das Reaktionsgemisch auf 500 g Eis und stellt den pH-Wert durch Zugabe von 5-n. Salzsäure auf ca.1. Dieses saure Gemisch wird 3-mal mit Methylenchlorid extrahiert, und die Extrakte zweimal mit 2-n. Salzsäure und 3-mal mit Wasser gewaschen. Die vereinigten Extrakte

trocknet man über Natriumsulfat und dampft das Lösungsmittel im Wasserstrahlvakuum ab. Dabei erhält man spontan kristallisierendes Rohprodukt vom Smp. 154-156°. Durch Umkristallisation aus Methylenchlorid/Hexan erhält man das 3-(2,3-Dimethyl-7-brom-5-benzofuranyl)-1,5-pentandiyl-dimethansulfonat vom Smp. 158-159° als weisse Kristalle.

Beispiel 22

Analog Beispiel 20 werden 3,81 g (0,01 Mol) 1-Benzyl-4-(2,3-dimethyl-7-cyano-5-benzofuranyl)-piperidin-hydrochlorid in 100 ml Dioxan (wasserfrei) gelöst und in Gegenwart von 0,38 g 5 %-igem Palladium-Kohle-Katalysator bei Raumtemperatur während 11 Stunden unter Normaldruck hydriert. Danach wird der Katalysator über Diatomeenerde abfiltriert und das Filtrat am Wasserstrahlvakuum eingedampft. Das erhaltene Rohprodukt kristalllisiert man zweimal aus Methanol/Aether um und erhält so das 4-(2,3-Dimethyl-7-cyano-5-benzofuranyl)-piperidin-hydrochlorid vom Smp. 182-184°.

Der Ausgangsstoff wird wie folgt hergestellt:

a) 3,08 g (0,01 m) 4-(2,3-Dimethyl-7-brom-5-benzofuranyl)-piperidin (vgl. Beispiel 21) werden in 100 ml Tetrahydrofuran gelöst und die Lösung mit 1,4 ml Triäthylamin und danach mit 1,30 ml Benzylbromid (0,0105 Mol) versetzt. Dabei entsteht ein weisser Niederschlag. Das Reaktionsgemisch wird 3 1/2 Stunden bei Raumtemperatur gerührt. Dann entfernt man das Lösungsmittel und überschüssiges Triäthylamin im Wasserstrahlvakuum und gibt den Rückstand auf Wasser. Man extrahiert 3-mal mit Aether und wäscht die vereinigten Aetherlösungen einmal mit 2-n. Natronlauge und anschliessend zweimal mit Wasser und einmal mit gesättigter Natriumchloridlösung. Dann trocknet man die vereinigten Aetherlösungen über Natriumsulfat und dampft sie im Wasserstrahlvakuum vollständig ein. Dabei fällt das bei 86-91° schmelzende 1-Benzyl-4-(2,3-dimethyl-7-brom-5-benzofuranyl)-piperidin an. Im Dünnschichtchromatogramm auf Silikagel im Laufmittel (Chloroform/5 % Methanol) zeigt dieses Produkt einen Rf-Wert von 0,4. Eine aus Aether/Hexan umkristallisierte Probe zeigt einen Smp. von 88-91°. Das Produkt ist für die Weiterverwendung von genügender Reinheit.

b) 3,98 g (0,01 Mol) 1-Benzyl-4-(2,3-dimethyl-7-brom-5-benzofuranyl)-piperidin und 0,95 g (0,011 Mol) Kupfer(I)-cyanid werden in 22,8 ml 1-Methyl-2-pyrrolidinon suspendiert und während 5 1/2 Stunden unter Stickstoff auf 200° erhitzt. Das schwarze Reaktionsgemisch wird abgekühlt und auf das Gemisch von 140 ml 50 %-iger wässriger Aethylendiaminlösung und ca. 100 g Eis gegossen. Dabei entsteht eine tiefblaue wässrige Lösung. Man extrahiert diese 3-mal mit Methylenchlorid, wäscht die organischen Phasen zweimal mit 50 %-iger wässriger Aethylendiaminlösung und anschliessend 4-mal mit Wasser und einmal mit gesättigter Natriumchloridlösung neutral. Die vereinigten organischen Extrakte trocknet man über Natriumsulfat und dampft sie nach Zusatz von ca. 50 ml Toluol im Wasserstrahlvakuum ein. Man erhält dabei ein braun-schwarzes Oel, das an der 30-fachen Menge Kieselgel mit Chloroform als Lösungs- und Eluiermittel chromatographiert wird. Die dabei anfallende Rohbase wird in Methanol mit ätherischer Chlorwasserstofflösung ins Hydrochlorid übergeführt und dieses mit Aether zur Kristallisation gebracht. Das rohe Hydrochlorid kristallisiert man aus Methanol/Aether um und erhält so reines 1-Benzyl-4-(2,3-dimethyl-7-cyano-5-benzofuranyl)-piperidin-hydrochlorid vom Smp. 186-190°.

Beispiel 23

Analog Beispiel 8 erhält man durch Reduktion mittels Lithiumaluminiumhydrid aus 2,87 g (0,01 Mol) 1-Formyl-4-(2,3-dimethyl-7-methoxy-5-benzofuranyl)-piperidin das

1-Methyl-4-(2,3-dimethyl-7-methoxy-5-benzofuranyl)-piperidin und dessen Hydrochorid, aus 2,71 g (0,01 Mol) 1-Formyl-4-(2,3,7-trimethyl-5-benzofuranyl)-piperidin das 1-Methyl-4-(2,3,7-trimethyl-5-benzofuranyl)-piperidin und dessen Hydrochlorid, aus 3,13 g (0,01 Mol) 1-Formyl-4-(4-methoxy-6,7,8,9-tetrahydro-2-dibenzofuranyl)-piperidin das 1-Methyl-4-(4-methoxy-6,7,8,9-tetrahydro-2-dibenzofuranyl) piperidin und dessen Hydrochlorid, und aus 2,97 g (0,01 Mol) 1-Formyl-4-(4-methyl-6,7,8,9-tetrahydro-2-dibenzofuranyl)-piperidin das 1-Methyl-4-(4-methyl-6,7,8,9-tetrahydro-2-dibenzofuranyl)-piperidin und dessen Hydrochlorid

Die als Ausgangsstoffe benötigten 1-Formyl-Verbindungen werden aus den entsprechenden, in den Beispielen 17, 19 bzw. 20 beschriebenen Verbindungen ohne 1-Substituent analog Beispiel 8 a) hergestellt.

Beispiel 24

Analog Beispiel 14 erhält man unter Verwendung

von 2,59 g (0,010 Mol) 4-(2,3-Dimethyl-7-methoxy-5-benzofuranyl)-piperidin (vgl. Beispiel 17) das 1-(2-Propinyl)-4-(2,3-dimethyl-7-methoxy-5-benzofuranyl)-piperidin und sein Hydrochlorid,

von 2,43 g (0,010 Mol) 4-(2,3,7-Trimethyl-5-benzofuranyl)-piperidin (vgl. Beispiel 20) das 1-(2-Propinyl)-4-(2,3,7-trimethyl-5-benzofuranyl)-piperidin und sein Hydrochlorid,

von 3,08 g (0,010 Mol) 4-(2,3-Dimethyl-7-brom-5-benzofuranyl)-piperidin (vgl. Beispiel 21) das 1-(2-Propinyl)-4-(2,3-dimethyl-7-brom-5-benzofuranyl)-piperidin und sein Hydrochlorid,

von 2,56 g (0,010 Mol) 4-(2,3-Dimethyl-7-cyano-5-benzofuranyl)-piperidin (vgl. Beispiel 22) das 1-(2-Propinyl)-4-(2,3-dimethyl-7-cyano-5-benzofuranyl)-piperidin und sein Hydrochlorid,

von 2,85 g (0,010 Mol) 4-(4-Methoxy-6,7,8,9-tetrahydro-2-dibenzofuranyl)-piperidin das 4-(2-Propinyl)-4-(4-Methoxy-6,7,8,9-tetrahydro-2-dibenzofuranyl)-piperidin und sein Hydrochlorid, und

von 2,69 g (0,010 Mol) 4-(4-Methyl-6,7,8,9-tetrahydro-2-dibenzofuranyl)-piperidin das 4-(2-Propinyl)-4-(4-methyl-6,7,8,9-tetrahydro-2-dibenzofuranyl)-piperidin und sein Hydrochlorid.

Beispiel 25

Analog Beispiel 14 erhält man durch Umsetzung von 2,29 g (0,010 Mol) 4-(2,3-Dimethyl-5-benzofuranyl)-piperidin (vgl. Beispiel 1) mit 1,33 g (0,011 Mol) Allylbromid das 1-Allyl-4-(2,3-dimethyl-5-benzofuranyl)-piperidin und sein Hydrochlorid,

und durch Umsetzung von 2,55 g (0,010 Mol) 4-(6,7,8,9-tetrahydro-2-dibenzofuranyl)-piperidin (vgl. Beispiel 8) mit 1,33 g (0,011 Mol) Allylbromid das 1-Allyl-4-(6,7,8,9-tetrahydro-2-dibenzofuranyl)-piperidin und sein Hydrochlorid.

Beispiel 26

Analog Beispiel 14 erhält man durch Umsetzung von 2,29 g (0,010 Mol) 4-(2,3-Dimethyl-5-benzofuranyl)-piperidin (vgl. Beispiel 1) mit 1,49 g (0,011 Mol) Cyclopropylmethylbromid das 1-(Cyclopropylmethyl)-4-(2,3-dimethyl-5-benzofuranyl)-piperidin und sein Hydrochlorid,

und durch Umsetzung von 2,55 g (0,010 Mol) 4-(6,7,8,9-tetrahydro-2-dibenzofuranyl)-piperidin (vgl Beispiel 8) mit 1,49 g (0,011 Mol) Cyclopropylmethylbromid das 1-(Cyclopropylmethyl)-4-(6,7,8,9-tetrahydro-2-dibenzofuranyl)-piperidin und sein Hydrochlorid.

Beispiel 27

2,59 g (0,01 Mol) 4-(2,3-Dimethyl-7-methoxy-5-benzofuranyl)-piperidin und 2,45 g (0,05 Mol) Natriumcyanid werden in 13 ml wasserfreiem Dimethylsulfoxid gelöst und unter Stickstoff 15 Stunden unter Rückfluss gekocht. [Methode von James R. McCarthy et al., Tetrahedron Letters 1978, 5183] Danach lässt man das Reaktionsgemisch auf Raumtemperatur erkalten, stellt durch Zugabe von 2-n. Salzsäure (Achtung auf die Entwicklung von Cyanwasserstoff) den pH-Wert des Reaktionsgemisches auf 4 bis 5 ein und extrahiert mit Aethylacetat. Die Extrakte trocknet man über Magnesiumsulfat und dampft die Lösungsmittel im Wasserstrahlvakuum ab. Das erhaltene Rohprodukt wird in Methanol mit Chlorwasserstoff ins Hydrochlorid übergeführt. Dieses wird durch 2-malige Umkristallisation aus Methanol-Aether gereinigt, wobei man das 4-(2,3-Dimethyl-7-hydroxy-5-benzofuranyl)-piperidin-hydrochlorid vom Smp. 168-173° erhält.

Patentansprüche

1. Tetrahydropyridin- und Piperidinderivate entsprechen der allgemeinen Formel I

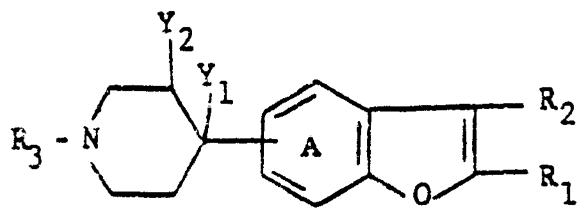

(I)

in welcher $R_1$ und $R_2$ unabhängig voneinander Wasserstoff oder Niederalkyl oder zusammen Niederalkylen, $R_3$ Wasserstoff, Niederalkyl, Niederalkenyl, Niederalkinyl, Cycloalkyl oder Cycloalkylniederalkyl und $Y_1$ und $Y_2$ je Wasserstoff oder zusammen eine zusätzliche Bindung bedeuten und der Ring A nicht weiter substituiert oder weiter substituiert ist, und deren Säureadditionssalze.

2. Verbindungen der im Anspruch 1 angegebenen allgemeinen Formel I, in denen $R_1$, $R_2$, $R_3$, $Y_1$ und $Y_2$ die im Anspruch 1 angegebene Bedeutung haben, und Ring A nicht weiter substituiert oder durch Niederalkyl, Niederalkoxy, Halogen bis Atomnummer 35, Cyano oder Hydroxy einfach weitersubstituiert ist, und deren Säureadditionssalze.

3. Verbindungen der im Anspruch 1 angegebenen allgemeinen Formel I, in denen $R_1$ Methyl, $R_2$ Wasserstoff oder Methyl, oder $R_1$ und $R_2$ zusammen Tetramethylen bedeuten, $R_3$, $Y_1$ und $Y_2$ die im Anspruch 1 angegebene Bedeutung haben, und Ring A nicht weiter substituiert oder durch Niederalkyl, Niederalkoxy, Halogen bis Atomnummer 35, Cyano oder Hydroxy weitersubstituiert ist, und deren Säureadditionssalze.

4. Verbindungen der im Anspruch 1 angegebenen allgemeinen Formel I, in denen $R_1$ Methyl, $R_2$ Methyl oder Wasserstoff, oder $R_1$ und $R_2$ zusammen Tetramethylen bedeuten, $R_3$ Wasserstoff oder einen im Anspruch 1 definierten Rest mit höchstens 4 Kohlenstoffatomen bedeutet, $Y_1$ und $Y_2$ die im Anspruch 1 unter der Formel I angegebene Bedeutung haben und der Ring A nicht weiter substituiert oder durch Niederalkyl, Niederalkoxy, Halogen bis Atomnummer 35 oder Cyano einfach weitersubstituiert ist, und deren Säureadditionssalze.

5. Verbindungen der im Anspruch 1 angegebenen allgemeinen Formel I, in denen $R_1$ Methyl, $R_2$ Methyl oder Wasserstoff, oder $R_1$ und $R_2$ zusammen Tetramethylen bedeuten, $R_3$ Wasserstoff, Niederalkyl mit höchstens 4 Kohlenstoffatomen, Allyl, 2-Propinyl, Cyclopropyl oder Cyclopropylmethyl bedeutet, $Y_1$ und $Y_2$ die unter der Formel I angegebene Bedeutung haben und der Ring A nicht weiter substituiert oder durch Methyl, Halogen bis Atomnummer 35 oder Cyano einfach weitersubstituiert ist, und deren Säureadditionssalze.

6. Verbindungen der allgemeinen Formel I, in denen $R_1$ und $R_2$ je Methyl oder zusammen Tetramethylen bedeuten, $R_3$ Wasserstoff, Methyl oder 2-Propinyl bedeutet und $Y_1$ und $Y_2$ je ein Wasserstoffatom bedeuten, Ring A nicht weiter substituiert oder durch Methyl, Methoxy, Halogen bis Atomnummer 35 oder Cyano einfach weitersubstituiert ist, und der stickstoffhaltige Ring in 5- oder 6-Stellung des Benzofuranringsystems steht, und deren Säureadditionssalze.

7. 4-(2,3-Dimethyl-5-benzofuranyl)-piperidin und dessen pharmazeutisch annehmbare Säureadditionssalze.

8. 4-(2,3-Dimethyl-6-benzofuranyl)-piperidin und dessen pharmazeutische annehmbare Säureadditionssalze.

9. 4-(6,7,8,9-Tetrahydro-2-dibenzofuranyl)-piperidin und dessen pharmazeutisch annehmbare Säureadditionssalze.

10. 1-Methyl-4-(2,3-dimethyl-5-benzofuranyl)-piperidin und dessen pharmazeutisch annehmbare Salze.

11. 4-(2,3-Dimethyl-4-benzofuranyl)-piperidin und dessen pharmazeutisch annehmbare Salze.

12. 4-(2,3-Dimethyl-7-benzofuranyl)-piperidin und dessen pharmazeutisch annehmbare Salze.

13. 1-Methyl-4-(2,3-dimethyl-6-benzofuranyl)-piperidin und dessen pharmazeutisch annehmbare Salze.

14. 4-(6,7,8,9-Tetrahydro-1-dibenzofuranyl)-piperidin und dessen pharmazeutisch annehmbare Salze.

15. 1-Methyl-4-(2-methyl-5-benzofuranyl)-1,2,3,6-tetrahydropyridin und dessen pharmazeutisch annehmbare Salze.

16. 1-Methyl-4-(2-methyl-5-benzofuranyl)-piperidin und dessen pharmazeutisch annehmbare Salze.

17. 4-(2-methyl-5-benzofuranyl)-piperidin und dessen pharmazeutisch annehmbare Salze.

18. 1-(2-Propinyl)-4-(2-methyl-5-benzofuranyl)-piperidin und dessen pharmazeutisch annehmbare Salze.

19. 1-(2-Propinyl)-4-(2,3-dimethyl-5-benzofuranyl)-piperidin und dessen pharmazeutisch annehmbare Salze.

20. 4-(2,3-Dimethyl-7-methoxy-5-benzofuranyl)-piperidin und dessen pharmazeutisch annehmbare Salze.

21. 1-Cyclopropyl-4-(2,3-dimethyl-6-benzofuranyl)-piperidin.

22. 4-(2,3,7-Trimethyl-5-benzofuranyl)-piperidin und dessen pharmazeutisch annehmbare Salze.

23. 4-(2,3-Dimethyl-7-brom-5-benzofuranyl)-piperidin und dessen pharmazeutisch annehmbare Salze.

24. 4-(2,3-Dimethyl-7-cyano-5-benzofuranyl)-piperidin und dessen pharmazeutisch annehmbare Salze.

25. 4-(2,3-Dimethyl-7-hydroxy-5-benzofuranyl)-piperidin und dessen pharmazeutisch annehmbare Salze.

26. Pharmazeutische Zusammensetzungen, gekennzeichnet durch einen Gehalt an einer Verbindung gemäss Anspruch 1 oder einem pharmazeutisch annehmbaren Säureadditionssalz derselben, und mindestens einem pharmazeutischen Trägerstoff.

27. Pharmazeutische Zusammensetzungen, gekennzeichnet durch einen Gehalt an einer Verbindung gemäss einem der Ansprüche 2 bis 25 oder einem pharmazeutisch annehmbaren Säureadditionssalz derselben, und mindestens einem pharmazeutischen Trägerstoff.

28. Verfahren zur Herstellung von Tetrahydropyridin- und Piperidinderivaten der im Anspruch 1 definierten Formel I und ihren Säureadditionssalzen, dadurch gekennzeichnet, dass man

a) in einer Verbindung der allgemeinen Formel II

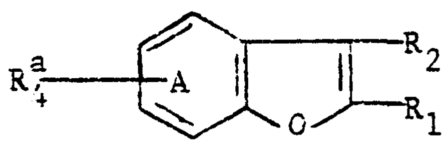

(II)

in welcher $R_4^a$ einen 1,2,3,6-Tetrahydro-4-pyridyl- oder 4-Piperidylrest bedeutet, welcher gegebenenfalls an mindestens einem der dem Stickstoffatom benachbarten Ringkohlenstoffatome durch einen durch zwei Wasserstoffatome ersetzbaren, zweiwertigen Rest $X_a$ substituiert ist und an dessen Stickstoffatom ein definitionsgemässer Rest $R_3$, der gegebenenfalls in seiner 1-Stellung durch einen definitionsgemässen Rest $X_a$ substituiert ist, oder ein einen definitionsgemässen Rest $X_a$ und Niederalkoxy tragendes Kohlenstoffatom gebunden ist, wobei insgesamt mindestens ein definitionsgemässer Rest $X_a$ vorhanden sein muss, $R_1$ und $R_2$ die unter der Formel I angegebene Bedeutung haben und der Ring A nicht weiter substituiert oder weiter substituiert ist, den oder die Reste $X_a$ und, falls vorhanden, das vorgenannte Niederalkoxy durch Wasserstoff ersetzt, oder

b) in einer Verbindung der allgemeinen Formel III

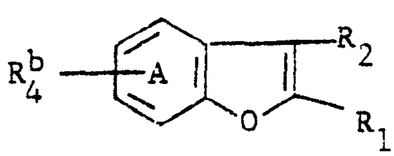

(III)

in welcher $R_4^b$ einen am gegebenenfalls quaternären Stickstoffatom den Rest $R_3$ tragenden, gegegebenenfalls partiell

hydrierten 4-Pyridylrest bedeutet, und $R_1$ und $R_2$ die unter der Formel I angegebene Bedeutung haben und der Ring A nicht weiter substituiert oder weiter substituiert ist, den Rest $R_4^b$ zu einem in 1-Stellung durch $R_3$ substituierten 1,2,3,6-Tetrahydro-4-pyridyl- oder 4-Piperidylrest reduziert, oder

c) in einer Verbindung der allgemeinen Formel IV

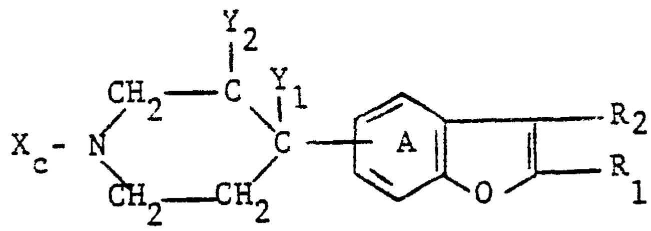

(IV)

in welcher $X_c$ einen durch Wasserstoff ersetzbaren Rest bedeutet und $R_1$, $R_2$, $Y_1$ und $Y_2$ die unter der Formel I angegebene Bedeutung haben und der Ring A nicht weiter substituiert oder weiter substituiert ist, den Rest $X_c$ durch Wasserstoff ersetzt, oder

d) aus einer Verbindung der allgemeinen Formel V

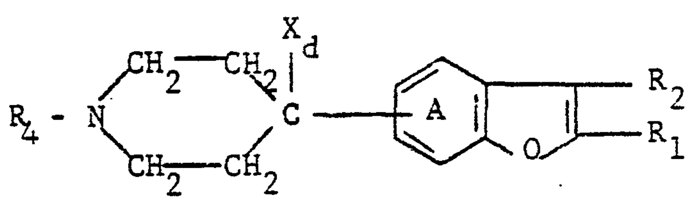

(V)

in welcher $X_d$ gegebenenfalls verestertes Hydroxy bedeutet und $R_1$, $R_2$ und $R_3$ die unter der Formel I angegebene Bedeutung haben und der Ring A nicht weiter substituiert oder weiter substituiert ist, die Verbindung der Formel

$$H - X_d \qquad (VI)$$

abspaltet, oder

e) in einer Verbindung der allgemeinen Formel VII

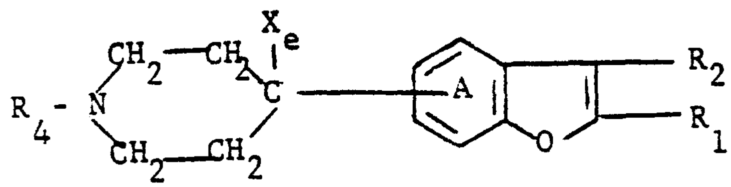

(VII)

in welcher $X_e$ einen durch Wasserstoff ersetzbaren Rest bedeutet und $R_1$, $R_2$ und $R_3$ die unter der Formel I angegebene Bedeutung haben, und der Ring A nicht weiter substituiert oder weiter substituiert ist, den Rest $X_e$ durch Wasserstoff ersetzt, oder

f) eine Verbindung der allgemeinen Formel VIII

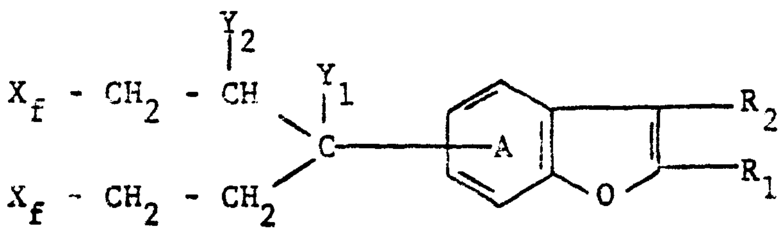

(VIII)

in welcher eines der Symbole $X_f$ die Gruppe $-NHR_3$, in welcher $R_3$ die unter der Formel I angegebene Bedeutung hat, und das andere eine reaktionsfähige veresterte Hydroxygruppe bedeutet, und $R_1$, $R_2$, $Y_1$ und $Y_2$ die unter der Formel I angegebene Bedeutung haben, und der Ring A nicht weiter substituiert oder weiter substituiert ist, cyclisiert, oder

g) eine Verbindung der allgemeinen Formel IX

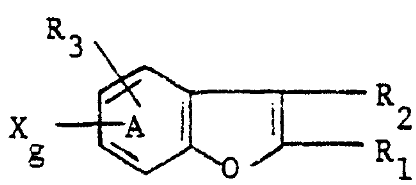

(IX)

und eine Verbindung der allgemeinen Formel X

0006524



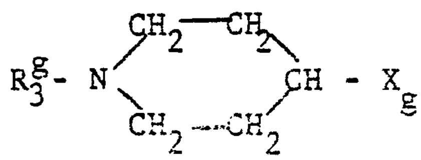

(X)

worin eines der Symbole $X_g$ einen Halogenomagnesiumrest oder einen Alkalimetallrest und das andere Halogen bedeutet, $R_3^g$ einen Rest entsprechend der unter der Formel I angegebenen Definition für $R_3$ mit Ausnahme von Wasserstoff bedeutet und $R_1$ und $R_2$ die unter der Formel I angegebene Bedeutung haben, und der Ring A nicht weiter substituiert oder weiter substituiert ist, miteinander umsetzt, oder

h) auf das Gemisch aus einer Verbindung der allgemeinen Formel XI

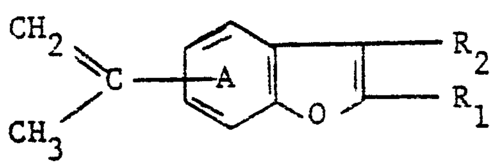

(XI)

in welcher $R_1$ und $R_2$ die unter der Formel I angegebene Bedeutung haben und der Ring A nicht weiter substituiert oder weiter substituiert ist, einer Verbindung der allgemeinen Formel XII,

$$R_3 - NH_2 \qquad (XII)$$

in welcher $R_3$ die unter der Formel I angegebene Bedeutung hat, und der mindestens doppeltmolaren Menge Formaldehyd, oder auf das aus den vorgenannten drei Reaktionskomponenten erhältliche rohe Reaktionsprodukt eine Protonensäure einwirken lässt, und/oder gewünschtenfalls in eine Verbindung der allgemeinen Formel I, in welcher $R_3$ Wasserstoff bedeutet und $R_1$, $R_2$, $Y_1$ und $Y_2$ die unter der Formel I angege-

bene Bedeutung haben, einen von Wasserstoff verschiedenen Rest $R_3$ einführt, und/oder gewünschtenfalls eine Verbindung der allgemeinen Formel I, in welcher der Ring A durch Brom substituiert und der Rest $R_3$ von Wasserstoff verschieden ist, während $R_1$, $R_2$, $Y_1$ und $Y_2$ die unter der Formel I angegebene Bedeutung haben, oder eine entsprechende Verbindung mit Jod anstelle von Brom, mit einem Metallcyanid umsetzt, und/oder gewünschtenfalls eine Verbindung der allgemeinen Formel I, in welcher der Ring A durch Niederalkoxy substituiert ist, während $R_1$, $R_2$, $R_3$, $Y_1$ und $Y_2$ die unter der Formel I angegebene Bedeutung haben, oder eine entsprechende Verbindung mit gegebenenfalls substituiertem Benzyloxy anstelle von Niederalkoxy, mit einem ätherspaltendem Mittel behandelt, und/oder gewünschtenfalls eine Verbindung der allgemeinen Formel I in anderer an sich bekannter Weise in eine andere Verbindung der allgemeinen Formel I umwandelt, und/oder ein erhältliches Isomerengemisch (Racematgemisch) in die reinen Isomeren (Racemate) auftrennt, und/oder ein erhältliches Racemat in die optischen Antipoden aufspaltet und/oder eine erhältliche freie Verbindung in ein Salz, oder ein erhaltenes Salz in die freie Verbindung oder in ein anderes Salz überführt.

29. Verwendung einer Verbindung gemäss einem der Ansprüche 1 bis 25 oder eines pharmazeutisch annehmbaren Salzes einer solchen als Antidepressivum.

0006524

Nummer der Anmeldung

Europäisches Patentamt

# EUROPÄISCHER RECHERCHENBERICHT

EP 79 101 887.2

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| | DE - A - 2 408 476 (CIBA-GEIGY)<br>* Ansprüche 1, 49 *<br>-- | 28,29 | C 07 D 405/04<br>A 61 K 31/445 |
| | DE - A - 2 537 837 (CIBA-GEIGY)<br>* Ansprüche 12, 20 *<br>-- | 28,29 | |
| | DE - A - 2 653 147 (CIBA-GEIGY)<br>* Ansprüche 1, 9 *<br>-- | 28,29 | RECHERCHIERTE SACHGEBIETE (Int. Cl.3) |
| | DE - A - 2 423 835 (CIBA-GEIGY)<br>* Anspruch 1 *<br>-- | 29 | A 61 K 31/445<br>C 07 D 405/04 |
| A | DE - A - 1 620 315 (LABORATOIRES LABAZ)<br>---- | | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 19-09-1979 | FROELICH |